# EUROPEAN PATENT SPECIFICATION

(11) **EP 4 226 157 B1**
(45) Date of publication and mention of the grant of the patent: **03.12.2025**
(21) Application number: 21798574.6
(22) Date of filing: 08.10.2021
(51) Int. Cl.: G01N 33/569

(54) **METHOD FOR DETERMINING WHETHER OR NOT VIRUS-NEUTRALIZING ANTIBODIES ARE PRESENT AND IN VITRO METHOD FOR SCREENING COMPOUNDS FOR THEIR ABILITY TO NEUTRALIZE A VIRUS**
VERFAHREN ZUR BESTIMMUNG DES VORHANDENSEINS BZW. NICHT VIRUSNEUTRALISIERENDER ANTIKÖRPER UND IN-VITRO-VERFAHREN ZUM SCREENING VON VERBINDUNGEN AUF IHRE VIRUSNEUTRALISIERUNGSFÄHIGKEIT
MÉTHODE POUR DÉTERMINER SI DES ANTICORPS NEUTRALISANT DES VIRUS SONT PRÉSENTS OU NON ET MÉTHODE IN VITRO DE CRIBLAGE DE COMPOSÉS POUR LEUR CAPACITÉ À NEUTRALISER UN VIRUS

(30) Priority: 09.10.2020 LU 102116
(43) Date of publication of application: 16.08.2023
(73) Proprietor: Helmholtz Zentrum München - Deutsches Forschungszentrum für Gesundheit und Umwelt (GmbH), 85764 Neuherberg (DE); Eximmium Biotechnologies GmbH, 81377 Munich (DE)
(72) Inventor: HAMMERSCHMIDT, Wolfgang, 80686 München (DE); PICH, Dagmar, 81245 München (DE); ZEIDLER, Reinhard, 82140 Olching (DE)
(74) Representative: Schiweck Weinzierl Koch Patentanwälte Partnerschaft mbB
(86) International application number: PCT/EP2021/077818
(87) International publication number: WO 2022/074180

(56) References cited:
- AZAD TAHA ET AL: "Nanoluciferase complementation-based bioreporter reveals the importance of N-linked glycosylation of SARS-CoV-2�S for viral entry", MOLECULAR THERAPY, vol. 29, no. 6, 1 June 2021 (2021-06-01), US, pages 1984 - 2000, XP55862562, ISSN: 1525-0016, Retrieved from the Internet <URL:https://www.ncbi.nlm.nih.gov/pmc/articles/PMC7872859/pdf/main.pdf> DOI: 10.1016/j.ymthe.2021.02.007
- AZAD TAHA ET AL: "SARS-CoV-2 S1 NanoBiT: A nanoluciferase complementation-based biosensor to rapidly probe SARS-CoV-2 receptor recognition", BIOSENSORS AND BIOELECTRONICS, vol. 180, 2 March 2021 (2021-03-02), XP55862555, Retrieved from the Internet <URL:https://www.ncbi.nlm.nih.gov/pmc/articles/PMC7921772/pdf/main.pdf>
- HU JIE ET AL: "Development of cell-based pseudovirus entry assay to identify potential viral entry inhibitors and neutralizing antibodies against SARS-CoV-2", vol. 7, no. 4, 17 July 2020 (2020-07-17), NL, pages 551 - 557, XP055793168, ISSN: 2352-3042, Retrieved from the Internet <URL:https://www.ncbi.nlm.nih.gov/pmc/articles/PMC7366953/pdf/main.pdf> DOI: 10.1016/j.gendis.2020.07.006
- ZETTL FERDINAND ET AL: "Rapid Quantification of SARS-CoV-2-Neutralizing Antibodies Using Propagation-Defective Vesicular Stomatitis Virus Pseudotypes", VACCINES, vol. 8, no. 3, 15 July 2020 (2020-07-15), pages 386, XP055793173, DOI: 10.3390/vaccines8030386
- SAEED M F ET AL: "Novel, rapid assay for measuring entry of diverse enveloped viruses, including HIV and rabies", JOURNAL OF VIROLOGICAL METHODS, ELSEVIER BV, NL, vol. 135, no. 2, 1 August 2006 (2006-08-01), pages 143 - 150, XP027892237, ISSN: 0166-0934, [retrieved on 20060801]
- TSCHERNE D M ET AL: "An enzymatic virus-like particle assay for sensitive detection of virus entry", JOURNAL OF VIROLOGICAL METHODS, ELSEVIER BV, NL, vol. 163, no. 2, 1 February 2010 (2010-02-01), pages 336 - 343, XP026824813, ISSN: 0166-0934, [retrieved on 20091029]
- WOLF MIKE C ET AL: "A catalytically and genetically optimized Î2-lactamase-matrix based assay for sensitive, specific, and higher throughput analysis of native henipavirus entry characteristics", VIROLOGY JOURNAL, BIOMED CENTRAL, LONDON, GB, vol. 6, no. 1, 31 July 2009 (2009-07-31), pages 119, XP021059646, ISSN: 1743-422X, DOI: 10.1186/1743-422X-6-119
- SASAKI MICHIHITO ET AL: "Development of a rapid and quantitative method for the analysis of viral entry and release using a NanoLuc luciferase complementation assay", VIRUS RESEARCH, AMSTERDAM, NL, vol. 243, 23 October 2017 (2017-10-23), pages 69 - 74, XP085278271, ISSN: 0168-1702, DOI: 10.1016/J.VIRUSRES.2017.10.015
- SPITZER DIRK ET AL: "Green Fluorescent Protein-Tagged Retroviral Envelope Protein for Analysis of Virus-Cell Interactions", vol. 77, no. 10, 15 May 2003 (2003-05-15), US, pages 6070 - 6075, XP055793629, ISSN: 0022-538X, Retrieved from the Internet <URL:https://www.ncbi.nlm.nih.gov/pmc/articles/PMC154001/pdf/0863.pdf> DOI: 10.1128/JVI.77.10.6070-6075.2003
- ESTHER NOLTE-T HOEN ET AL: "Extracellular vesicles and viruses: Are they close relatives?", PROCEEDINGS OF THE NATIONAL ACADEMY OF SCIENCES, vol. 113, no. 33, 18 July 2016 (2016-07-18), pages 9155 - 9161, XP055609891, ISSN: 0027-8424, DOI: 10.1073/pnas.1605146113
- AZAD TAHA ET AL: "SARS-CoV-2 S1 NanoBiT: A nanoluciferase complementation-based biosensor to rapidly probe SARS-CoV-2 receptor recognition", BIOSENSORS AND BIOELECTRONICS, vol. 180, 2 March 2021 (2021-03-02), XP055862555, Retrieved from the Internet <URL:https://www.ncbi.nlm.nih.gov/pmc/articles/PMC7921772/pdf/main.pdf>
- AZAD TAHA ET AL: "Nanoluciferase complementation-based bioreporter reveals the importance of N-linked glycosylation of SARS-CoV-2 S for viral entry", MOLECULAR THERAPY, vol. 29, no. 6, 1 June 2021 (2021-06-01), US, pages 1984 - 2000, XP055862562, ISSN: 1525-0016, Retrieved from the Internet <URL:https://www.ncbi.nlm.nih.gov/pmc/articles/PMC7872859/pdf/main.pdf> DOI: 10.1016/j.ymthe.2021.02.007

## Description

### TECHNICAL FIELD OF THE INVENTION

The present invention provides a method for determining whether or not virus-neutralizing antibodies are present in a sample obtained from a subject. The present invention further relates to an *in vitro* method for screening (a) compound(s) for its/ their ability to neutralize a virus.

### BACKGROUND ART

Sera of virus-infected individuals can contain virus-specific antibodies, which are important diagnostic molecules. The functions of these antibodies may vary, but the biologically most relevant are so called "neutralizing antibodies" that can neutralize viral infectivity and thus directly contribute to viral clearance. Virus diagnostics also includes the quantitation of such virus-neutralizing antibodies, which is done in specialized laboratories all over the world.

However, assays for the measurement of virus-neutralizing antibodies require elaborate cell culture models and are tedious, time-consuming tasks. All standard assays require the handling of infectious virus and, depending on the virus type, read-outs that build on cytopathic effects (CPEs). CPEs can appear as plaques in monolayers of cultured cells that need to be susceptible or permissive to virus infection and may support replication of the virus, however, it is not always necessary for a virus to replicate to cause CPEs. CPEs can also be observed in virus-infected cells that do not support viral replication. For example, certain viruses induce cell fusions in culture within hours post infection. This is due to a massive fusogenic activity of the incoming virus that causes fusion between neighboring cells. Another type of CPE is immediate cell death after infection, a phenomenon that can have several causes. One cause is a massive release of interferons, but viruses can also cause acute apoptosis. The difference between these CPEs and convential, i.e. round areas of dead or vanished cells, is that only the latter can be counted faithfully. Regular plaque formation depends mostly on local spread of virus from actively virus-reproducing cells. This is why the infected cells are overlayered with low melting point agarose or carboxy-methyl cellulose to avoid long-range diffusion of virus progeny and, as a consequence, unclear i.e. diffuse plaque formation. Conventional CPEs can be counted directly by eye or indirectly using a microscope, but CPEs require several days after viral infection to become visible. Many viruses do not readily produce CPEs, which precludes a direct assessment of cell foci with virally infected cells. Most often, classical, conventional CPEs can only be seen when one uses suitable cells. The most prominent recent example is SARS-CoV-2. For its (difficult to spot) plaques, one has to use a variant of Vero cells that proliferate considerably slower than parental Vero cells.

Alternative methods exist, but they are even more cumbersome, because viral proteins in infected cells need to be visualized histochemically or virus progeny needs to be detected and quantitated by PCR techniques, for example. This standard approach and its many versions are functional and are used in clinical laboratories, but require specialists, e.g. virologists or trained technicians, and qualified material, such as cell lines, virus stocks, control antibodies, reagents, etc. Moreover, only certain laboratories can perform such tests, because a certification is obligatory according to national laws to handle infectious agents (Infektionsschutzgesetz in Germany). As viruses of interest are human pathogens or are potentially harmful, the laboratories must provide specialized equipment (as least biosafety level 2 safety cabinets, for example) and lab infrastructure (for example, dedicated certified rooms, thermal inactivation of infectious materials in autoclaves) to handle the viruses and virally infected cells safely and to eliminate contaminated materials, respectively. The problem of handling very dangerous viruses in neutralization assays has been recognized by many virologists. A workaround are retroviruses or lentiviruses that are pseudotyped with glycoproteins of viruses of interest. The retroviruses or lentiviruses (or other viral vectors) contain recombinant genomes that express marker proteins (GFP, luciferase, etc.) in target cells. Upon successful infection and expression of the marker, infectivity of the viral vectors can be measured. Such viral vectors are commercially available, but they do not solve the problem of handling infectious agents. Even worse, such potentially harmful viruses and vectors classify as genetically modified organisms (GMOs) that require additional safety measures and legal authorization for their handling. Neutralization assays with these viral vectors are lengthy, because the vector-transduced cells need to de novo express the marker protein in the infected cells to trace viral infection. Moreover, such viral vectors do not tolerate the incorporation of all heterologous glycoproteins or combinations of glycoproteins, because viral glycoproteins from viruses of interest often interfere with the assembly of the viral vector.

Additionally, virus neutralizing assays with patients' sera are difficult to perform, are expensive and take days before the results are obtained and can be communicated to clinicians. Moreover, the assays need manual steps and specialized handling that cannot be easily replaced by robots or dedicated automated analytical instruments.

As a matter of fact, neutralizing assays are further up to now not compatible with high-throughput screens. On the contrary, the expectation of clinicians to obtain such important information is high, because the detection of virus-neutralizing antibodies provides very valuable insights into the course of viral infection, its clinical prognosis or outcome and delivers critical information if patients have mounted robust, virus-specific immune responses or not. For example, in the current SARS-CoV-2 pandemic, it would be extremely valuable to know if a SARS-CoV-2 infected individual has mounted a solid immune response that can prevent reinfection with the virus later. Knowing whether an individual has mounted a high-quality immune response that is primarily characterized by virus-neutralizing antibodies and whether it is maintained for months or fades quickly, is of utmost importance for the patients, medical doctors, regulatory bodies and, eventually, for politicians.

Neutralizing antibody assays are also very informative when it comes to testing the efficacy of viral vaccines candidates or even vaccines that are on the market. This is because the level of neutralizing antibodies that can be reached by vaccination is a measure of success, efficacy of the vaccine and a proxy of protection from viral infection.

Additionally, under consideration of the prior art, the following has to be considered: Tscherne *et al.* (2010) and Wolf *et al.* (2009) both use EVs containing viral glycoproteins together with viral nucleoproteins (matrix protein of M1 of influenza virus or matrix protein VP40 of Ebola virus in Tscherne *et al.;* matrix protein M of Nipah virus in Wolf *et al.).* Tscherne *et al.* describe the use of antibodies in the context of documenting the specificity of their EVs/ VLPs, indicating that viral glycoproteins mediate the transfer of the label (ß-lactamase) to recipient/ target cells (and not another possible way of transfer), but Tscherne *et al.* lack to provide a virus neutralization test. The same applies for Wolf *et al.* not dealing with neutralizing antibodies.

Additionally, Tscherne *et al.* describe the usage of the influenza nucleoprotein M1 in a fusion with ß-lactamase. However, it turned out that the EV producing cells leak the fusion protein such that it was found as free protein in the supernatants of EV-producing cells, which is undesirable. Further, Hu et al. (Genes & Diseases, 2020, 7, 551-557) describes a cell-based pseudovirus entry assay to identify potential viral entry inhibitors, while Zettl et al. (Vaccines, 2020, 8, 386) is directed to the quantification of SARS-CoV-2-neutralizing antibodies using propagation-defective vesicular stromatitis. Saeed et al. (Journal of Virological Methods, 135, 2006, 143-150) instead describes an assay for measuring entry of diverse enveloped viruses, including HIV and rabies. For example, luciferase is directly packaged into HIV particles pseudotyped with envelope proteins of diverse viruses.

Thus, the prior art lacks to provide a reliable virus neutralization test to determine or quantify serum antibodies with neutralizing characteristics without said leakage-disadvantage.

The present invention aims at and adresses these needs described above.

### SUMMARY OF THE INVENTION

The above mentioned problems are solved by the subject-matter as defined in the claims. The invention is characterized by the appended claims.

The present invention provides a method for determining whether or not virus-neutralizing antibodies are present in a sample obtained from a subject, comprising the following steps:
- providing extracellular vesicles and a label, wherein the extracellular vesicles
   (i) are non-infectious,
   (ii) comprise one or more viral glycoprotein(s),
   (iii) are detectable via the label,
   (iv) are free of recombinant genetic material, and
   (v) comprise extracellular vesicle proteins that are incorporated into the membrane or the envelope of the extracellular vesicles, wherein a membrane protein in the extracellular vesicles is CD63;
- contacting the sample with said extracellular vesicles and cells, which are capable of taking up said extracellular vesicles, wherein the one or more viral glycoprotein(s) is/ are able to target a receptor of said cells and is/ are fusogenic, and
- determining whether or not said cells take up said extracellular vesicles;
   wherein a reduced uptake of said extracellular vesicles by said cells in comparison to a control, wherein said cells and said extracellular vesicles are contacted, but without said sample, is indicative of the presence of virus-neutralizing antibodies.

It is preferred for the method for determining whether or not virus-neutralizing antibodies are present in a sample obtained from a subject according to the present invention that the extracellular vesicles are detectable via the label, wherein the label is attached to an extracellular vesicle protein comprised in the extracellular vesicles.

It is further preferred for the method for determining whether or not virus-neutralizing antibodies are present in a sample obtained from a subject of the present invention that the extracellular vesicles are detectable via the label, wherein the label is attached to a viral capsid-protein or a viral nucleo-protein and wherein the one or more viral glycoprotein(s) and the viral capsid-protein or the viral nucleo-protein are from the same virus.

In one embodiment of the method for determining whether or not virus-neutralizing antibodies are present in a sample obtained from a subject of the present invention, the extracellular vesicles are detectable via the label, wherein the label is attached to the one or more viral glycoprotein(s) of the extracellular vesicles.

In one further embodiment of the method for determining whether or not virus-neutralizing antibodies are present in a sample obtained from a subject of the present invention, the extracellular vesicles are detectable via the label, wherein the label is attached to a viral tegument protein of the extracellular vesicles, and wherein the one or more viral glycoprotein(s) and the viral tegument protein are from the same virus.

In one embodiment of the method for determining whether or not virus-neutralizing antibodies are present in a sample obtained from a subject of the present invention, the label is a split protein, wherein a first part of the split protein is comprised in the extracellular vesicles and a second part of the split protein is comprised in the cells, and wherein the first and the second part of the split protein are able to form a complex.

The present invention further provides a method for determining whether or not virus-neutralizing antibodies are present in a sample obtained from a subject, comprising the following steps:
- providing a donor type of extracellular vesicles and a label, wherein the donor type of extracellular vesicles
   (i) are non-infectious,
   (ii) comprise one or more viral glycoprotein(s),
   (iii) are detectable via the label,
   (iv) are free of recombinant genetic material, and
   (v) comprise proteins that are incorporated into the membrane or the envelope of the extracellular vesicles, wherein a membrane protein in the extracellular vesicles is CD63;
- contacting the sample with said donor type of extracellular vesicles and a recipient type of extracellular vesicles, which are capable of taking up said donor type of extracellular vesicles, and
- determining whether or not said recipient type of extracellular vesicles take up said donor type of extracellular vesicles;
   wherein a reduced uptake of said donor type of extracellular vesicles by said recipient type of extracellular vesicles in comparison to a control, wherein said donor type of extracellular vesicles and said recipient type of extracellular vesicles are contacted, but without said sample, is indicative of the presence of virus-neutralizing antibodies.

The present invention is further directed to an *in vitro* method for screening (a) compound(s) for its/ their ability to neutralize a virus, comprising the following steps:
- providing extracellular vesicles and a label, wherein the extracellular vesicles
   (i) are non-infectious,
   (ii) comprise one or more viral glycoprotein(s),
   (iii) are detectable via the label,
   (iv) are free of recombinant genetic material, and
   (v) comprise proteins that are incorporated into the membrane or the envelope of the extracellular vesicles, wherein a membrane protein in the extracellular vesicles is CD63;
- contacting said compound(s) with said extracellular vesicles and cells, which are capable of taking up said extracellular vesicles, wherein the one or more viral glycoprotein(s) is/ are able to target a receptor of said cells and is/ are fusogenic, and
- determining whether or not said cells take up said extracellular vesicles;
   wherein a reduced uptake of said extracellular vesicles by said cells in comparison to a control, wherein said cells and said extracellular vesicles are contacted, but without said compound(s), is indicative of the ability of said compound(s) to neutralize said virus.

The present invention is further directed to an *in vitro* method for screening (a) compound(s) for its/ their ability to neutralize a virus, comprising the following steps:
- providing a donor type of extracellular vesicles and a label,
   wherein the extracellular vesicles
   (i) are non-infectious,
   (ii) comprise one or more viral glycoprotein(s),
   (iii) are detectable via the label,
   (iv) are free of recombinant genetic material, and
   (v) comprise proteins that are incorporated into the membrane or the envelope of the extracellular vesicles, wherein a membrane protein in the extracellular vesicles is CD63;
- contacting said compound(s) with said donor type of extracellular vesicles and a recipient type of extracellular vesicles, which are capable of taking up said donor type of extracellular vesicles, and
- determining whether or not said recipient type of extracellular vesicles take up said donor type of extracellular vesicles;
wherein a reduced uptake of said donor type of extracellular vesicles by said recipient type of extracellular vesicles in comparison to a control, wherein said donor type of extracellular vesicles and said recipient type of extracellular vesicles are contacted, but without said compound(s), is indicative of the ability of said compound(s) to neutralize said virus.

### BRIEF DESCRIPTION OF THE DRAWINGS

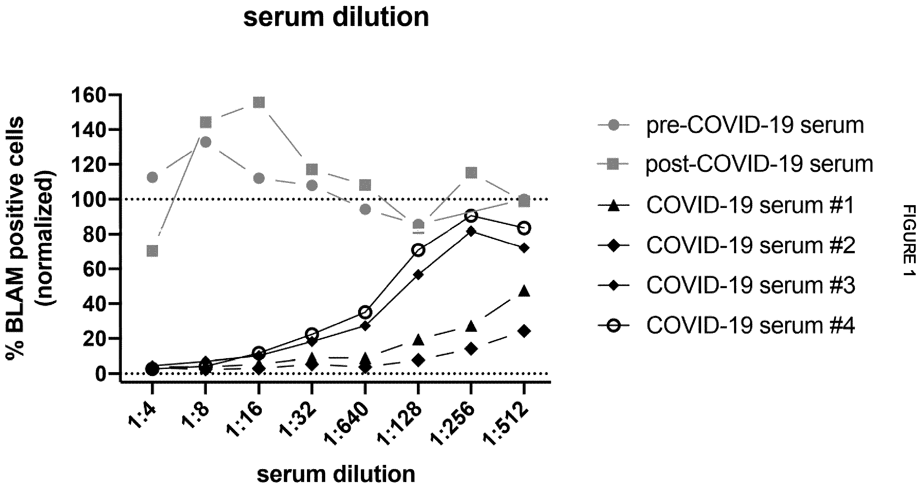

Figure 1 shows the results for the method for determining whether or not virus-neutralizing antibodies are present in a sample obtained from a subject of the present invention with six sera and EVs that carry the Spike protein of SARS-CoV-2 and a fusion protein consisting of CD63 and the prokaryotic enzyme ß-lactamase. A selected clone of Vero cells expressing the viral receptor ACE2 was used as target cells. Four sera were from patients who recovered from a severe form of COVID-19 (four lines and marks from the bottom) showing considerable neutralizing potential. Two sera were from one individual prior to (upper line with squares) and after (upper line with circles) a mild episode of COVID-19, with having only a weak neutralizing capacity.

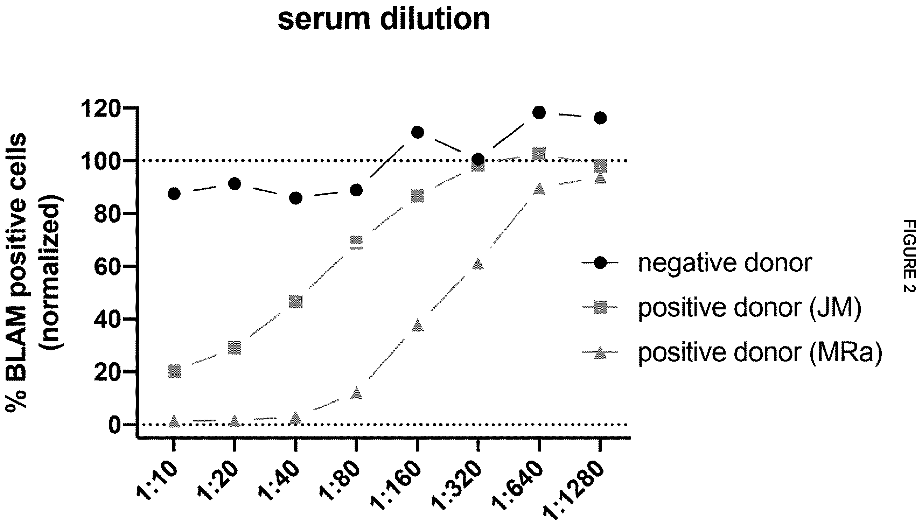

Figure 2 shows the results for the method for determining whether or not virus-neutralizing antibodies are present in a sample obtained from a subject of the present invention with three sera and EVs that carry all glycoproteins of Epstein-Barr virus (EBV) and a fusion protein consisting of CD63 and the prokaryotic enzyme ß-lactamase. CD19 positive B-lymphocytes contained in PBMCs served as target cells. One serum from an EBV-negative individual (circles) showed no neutralizing potential, whereas two sera from one individual with no episode of acute EBV infection (squares) and from an individual with chronic multiple sclerosis (triangles) showed neutralization of EBV. Both individuals were EBV-positive as determined by other means.

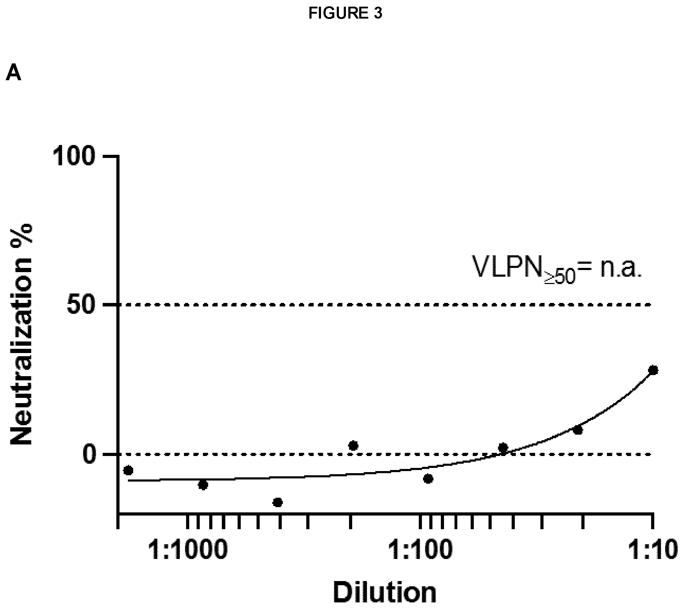

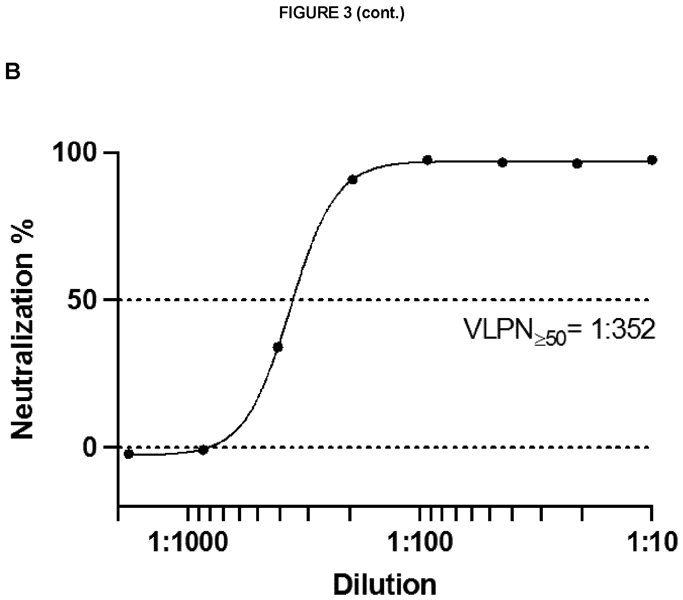

Figure 3 shows characteristic neutralization curves of two donors. Serum of a naïve donor (Figure 3A) showed no neutralization, even at high concentrations. Serum of an individual who has been vaccinated with a SARS-CoV-2 vaccine (Figure 3B) showed high neutralization. The extent of neutralization, termed titer of neutralizing antibodies, could be determined by calculating the dilution at which 50 % neutralization occurred. VLPN = Virus-like particle neutralization. The data were obtained with extracellular vesicles carrying the Spike protein of SARS-CoV-2 (Wuhan D614G strain) and a split label fused to the carboxyterminus of CD63. Recipient U251MG cells were engineered to express the SARS-CoV-2 receptor ACE2 and a split label consisting of an N-myristoylated split luciferase label. Serial dilutions of the sera were incubated with the extracellular vesicles for 30 min, the mixture was transferred to recipient cells cultivated in wells of a 96-well cluster plate and incubated for 4 hours. After removal of the supernatant, substrate was added to the cells, which were measured in a luminometer 2 min after substrate addition.

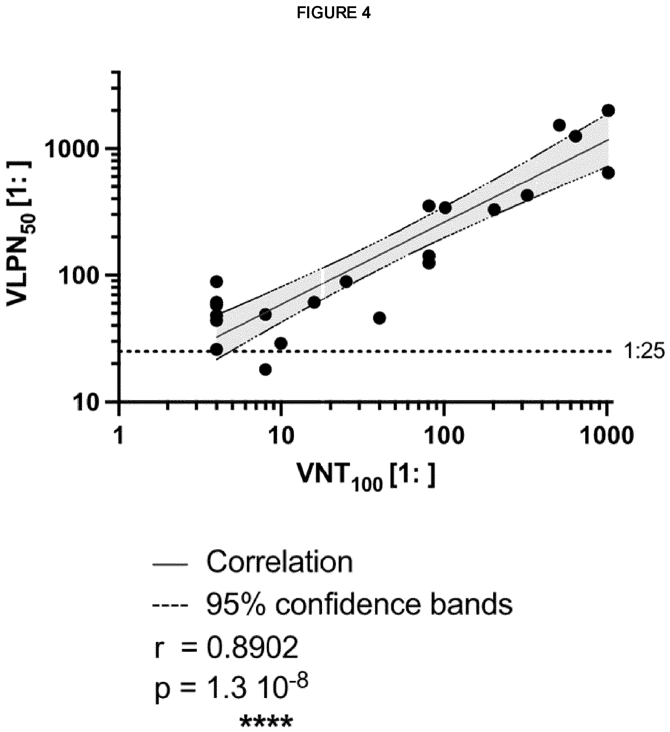

Figure 4 shows the validation of the novel Virus-like particle neutralization test (VLPNT) comparing 23 sera from COVID-19 patients analyzed in the VLPNT. The identical sera were also analyzed using the conventional Virus neutralization test (cVNT) in a BSL3 laboratory with replication competent SARS-CoV-2 virus in a plaque reduction assay. The results from both tests correlated very well according to statistical analyses provided. The results of the VLPNT were generated using the methodology described in the legend of Figure 3.

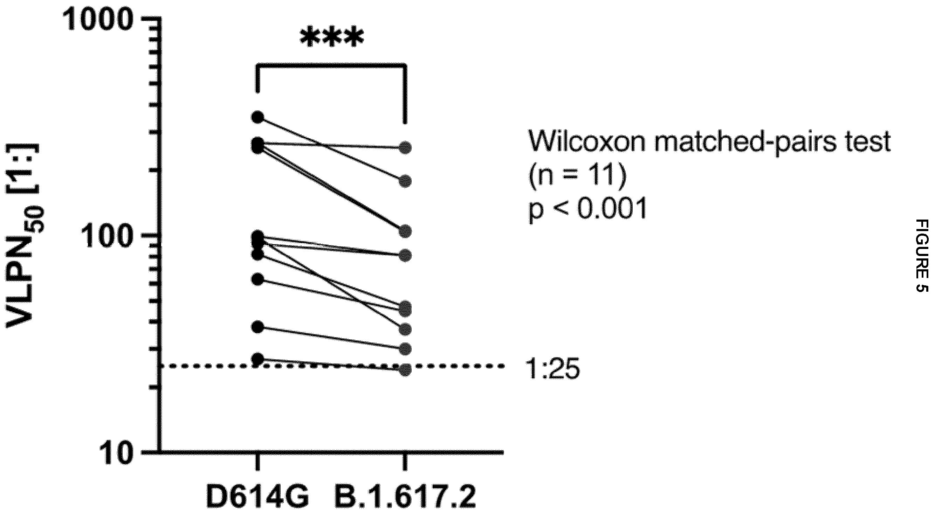

Figure 5 shows the adaption of the Virus-like particle neutralization test with two variants of concern (VOC) of SARS-CoV-2 (Wuhan D614G versus B.1.617.2) and a comparison of neutralizing serum titers as in Figure 3 with sera from vaccinees. The VLPNT could be easily modified to analyze the predominant mutant of the Spike protein of current SARS-CoV-2 field isolates. In line with the literature, neutralizing antibody titers of vaccinees were less potent in neutralizing the SARS-CoV-2 δ-mutant (B.1.617.2) compared with the D614G Spike mutant protein. The data were obtained with two different versions of extracellular vesicles carrying either the Spike protein of SARS-CoV-2 (Wuhan D614G strain) or the Spike protein of SARS-CoV-2 δ-mutant (B.1.617.2) and a split label fused to the carboxyterminus of CD63. Recipient U251MG cells were used, which express the SARS-CoV-2 receptor ACE2 and a split label consisting of an N-myristoylated split luciferase label. Serial dilutions of the sera were incubated with the extracellular vesicles for 30 min, the mixture was transferred to recipient cells cultivated in a 96-well cluster plate, which were incubated for 4 hours. After removal of the supernatant, substrate was added to the cells, which were measured in a luminometer after 2 min.

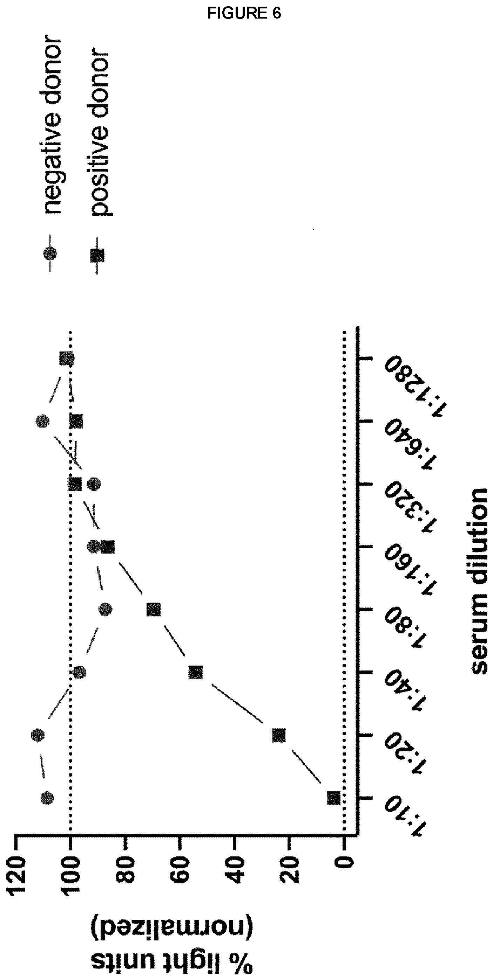

Figure 6 shows the results with sera from two individuals, a naïve human donor and a donor, who has been vaccinated with a SARS-CoV-2 vaccine. The sera were analyzed in the cell-free virus neutralization test using two types of extracellular vesicles. The donor type of extracellular vesicles carried the Spike protein of SARS-CoV-2 (Wuhan D614G strain) and the split protein label fused to the carboxyterminus of CD63. The recipient type of extracellular vesicles carried the ACE2 receptor and the corresponding split protein label, which was also fused to CD63. The donor type of extracellular vesicles was harvested from the supernatants of 293T cells that had been transfected with expression plasmids encoding the Spike protein and the split label fused to CD63. The recipient type of extracellular vesicles was harvested from U251MG cells stably transduced with retroviral expression vectors encoding ACE2 and the corresponding split label protein. Defined aliquots of supernatant harvested directly from the transfected 293T cell culture, containing the donor type of extracellular vesicles, was used and incubated with serial dilutions of serum as indicated for 30 min. Extracellular vesicles of the recipient type were purified and concentrated from supernatants of retrovirally transduced U251MG cells. Defined aliquots of the recipient type of extracellular vesicles were added to the serum dilutions followed by an incubation period of 4 hours. The extracellular vesicles were concentrated using magnetic beads and the results were obtained with the aid of a luminometer after incubating the beads with substrate for 2 min. The y-axis depicts the relative amount (percentage) of light units using a split label approach with internal test standards to delineate the range of the assay (0 and 100 %). The x-axis shows serum dilutions.

### DETAILED DESCRIPTION OF THE INVENTION

The inventors of the present invention provide respective methods for determining whether or not virus-neutralizing antibodies are present in a sample obtained from a subject and *in vitro* methods for screening compounds for their ability to neutralize a virus, wherein the needed materials are not infectious or contagious and can be handled in standard laboratories. Moreover, the methods of the present invention does not require a permission to work with infectious pathogens as the EVs contain no genetic information and thus cannot propagate infection.

This distinguishes the methods of the present invention as described herein from other virus neutralization tests of the prior art, which have the disadvantage to work with pseudotyped viruses, such as retroviral vectors, lentiviral vectors, vesicular stomatitis virus and many others. These virus neutralization tests of the prior art require a genetic viral component, which is packaged into the virus-like particles, i.e., EVs equipped with viral glycoproteins to allow their fusion with recipient/ target cells. As a consequence, the viral genetic material is delivered in an infectious mode of action into the recipient cells, which is not the case according to the methods of the present invention as described herein.

Another consequence of this approach of the prior art is that genetic recombinant material from viral pathogens is contained in stocks of such pseudotyped viral vectors and is delivered to recipient/ target cells. Recombinant viral vectors are classified as genetically modified organisms (GMOs) in all first world countries. Production of GMOs and their use is regulated by national laws (Gentechnikgesetz in Germany and similar laws in other countries). The EVs used in the present invention instead are free of recombinant genetic material (i.e. free of recombinant viral DNA or RNA) such that the respective stocks of EVs do not score as GMOs. This distinguishes the methods of the present invention significantly from approaches that make use of pseudotyped viral vectors (also called pseudoviruses) as used in Hu *et al.* (which uses lentivirus), Zettl *et al.* (which uses vesicular stomatitis virus), Saeed *et al.* (which uses lentivirus) and Spitzer *et al.* (which uses retrovirus).

The present invention provides in a first aspect a method for determining whether or not virus-neutralizing antibodies are present in a sample obtained from a subject, comprising the following steps:
- providing extracellular vesicles and a label, wherein the extracellular vesicles
   (i) are non-infectious,
   (ii) comprise one or more viral glycoprotein(s),
   (iii) are detectable via the label,
   (iv) are free of recombinant genetic material, and
   (v) comprise extracellular vesicle proteins that are incorporated into the membrane or the envelope of the extracellular vesicles, wherein a membrane protein in the extracellular vesicles is CD63;
- contacting the sample with said extracellular vesicles and cells, which are capable of taking up said extracellular vesicles, wherein the one or more viral glycoprotein(s) is/ are able to target a receptor of said cells and is/ are fusogenic, and
- determining whether or not said cells take up said extracellular vesicles;
   wherein a reduced uptake of said extracellular vesicles by said cells in comparison to a control, wherein said cells and said extracellular vesicles are contacted, but without said sample, is indicative of the presence of virus-neutralizing antibodies.

As used herein, in the context of the present invention, the term "determining whether or not" means that it is evaluated, if the condition under investigation or under examination is present or if that condition is not present with regard to a subject. In the specific context of the method of the present invention, the condition under investigation is if virus-neutralizing antibodies are present or not.

In the context of the present invention, a "virus-neutralizing antibody" or "virus-neutralizing antibodies" is/ are an antibody/ antibodies that can neutralize viral infectivity and thus directly contribute(s) to viral clearance. In this connection, as used herein, the term "neutralizing" is defined as a process resulting in or leading to the inhibition or reduction of the ability of the respective virus to infect a host cell/ host cells, e.g. the respective virus is neutralized by binding of a respective compound or antibody to the virus cell or components of the virus cell. The effect of the neutralizing step or the neutralizing activity of said virus-neutralizing antibody/ antibodies can be measured by the method according to the present invention, so that a direct conclusion can be drawn about the presence or absence of virus-neutralizing antibodies. Virus-neutralizing antibodies can mediate virus neutralization, preventing the fusion of said extracellular vesicles with said cells, leading to a reduced uptake of said extracellular vesicles by said cells in comparison to a control, which can be then easily detected and quantitated.

In this method of the present invention, the sample may be a biological sample. Further, the biological sample may be a body fluid sample, such as a blood sample, an urine sample or a saliva sample. Further, the blood sample, the urine sample or the saliva sample may be a human blood sample, a human urine sample or a human saliva sample.

In the context of the present invention, the term "subject" means a human or an animal, wherein the animal may be an ape, a dog, a cat, a cow, a pig, a horse, a camel, a dromedary, a mouse, a rat, a rabbit, a sheep or a goat. In the method for determining whether or not virus-neutralizing antibodies are present in a sample obtained from a subject according to the present invention, the subject may be a human individual. Further, the subject may be a human individual or a patient, who has already been diagnosed with a virus-infection or is supposed to have a virus infection or is supposed to have had a virus infection. It may also be that the subject is a human/ human individual.

The term "cell" as used herein and in the context of the present invention, may be used interchangeably with "target cell" or "recipient cell". All theses terms comprise that the respective cell may be able to incorporate cargo or material or substances etc. from e.g. an extracellular vesicle. Further, the cell may be an extracellular vesicle. Mammalian cells release different types of vesicles, collectively termed extracellular vesicles (EVs). EVs contain cellular and viral microRNAs (miRNAs) with an apparent potential to deliver their miRNA cargo to recipient cells to affect the stability of individual mRNAs and the transcriptome. Thus, as used herein and in the context of the present invention, the term "extracellular vesicles" comprises all different types of vesicles in general being secreted by cells, e.g. mammalian cells and may be broadly classified into exosomes, microvesicles (MVs) and apoptotic bodies according to their cellular origin. Exosomes and microvesicles (MVs) are both released by healthy cells, although they differ in several aspects. Exosomes are nanometer-sized vesicles of endocytic origin that form by inward budding of the limiting membrane of multivesicular endosomes (MVEs). Thus, their size is equivalent to that of the intraluminal vesicle within MVEs (40-120 nm). Due to their endocytic origin, exosomes are commonly enriched in endosome-associated proteins, such as Rab GTPases, SNAREs, annexins, and flotillin. Some of these proteins (e.g. Alix and Tsg101) are normally used as exosome markers. Tetraspanins (e.g. CD63, CD81, CD9) are a family of membrane proteins known to cluster into microdomains at the plasma membrane. These proteins are abundant in exosomes and considered to be markers as well. However, MVs bud from the cell surface and their size may vary between 50 nm to 1,000 nm. Common protein markers used to define these MVs are selectins, integrins and the CD40 ligand. Both exosomes and MVs are known to facilitate intercellular communication processes between cells in close proximity as well as distant cells. Exosomes that are released by immune cells may act as antigen-presenting vesicles, stimulate antitumoral immune responses or induce tolerogenic effects to suppress inflammation. Tumor cells have also been shown to exploit EVs to contribute to their progression by inactivating T lymphocytes or natural killer cells as well as promoting differentiation of regulatory T lymphocytes to suppress immune reactions. MVs have been implicated in coagulation and inflammation. Platelet- and monocyte-derived MVs are capable of promoting the assembly of the enzyme complexes acting on the coagulation cascade, resulting in cell fusion events that may lead to thrombus formation. Furthermore, MVs may act as both anti-inflammatory and pro-inflammatory factors depending on the stimulus that generates them and the cell from which they are released. In both cases, interaction of MVs with the target cell leads to secretion of cytokines that modulate the inflammatory response. These are just a few of the physiological and pathological roles in which EVs have been observed to be involved in a number, which is continuously increasing.

EVs have no well-documented fusion activity, they bind to the surface of cells and when the EVs are taken up by these cells by receptor-mediated engulfment, for example, they end up in the endosomal-lysosomal compartment and are destroyed subsequently. EVs can be loaded with extra proteins, which can be transiently or stably expressed in the EV-producing cells. All enveloped viruses use viral glycoproteins on their envelopes to foster infection. Infection is a multistep process starting with viral adhesion, receptor binding, receptor-mediated uptake, and, eventually, a fusion of the viral envelope with the endosomal membrane such that the "content" of the viral particles is released into the cytoplasm of the infected cell to deliver the viral genetic information. Certain viruses also go the direct route and fuse their membrane directly with the plasma membrane of the cells. Depending on the complexity of the virus, a single viral glycoprotein can mediate all these steps of the infection process, but up to five different viral glycoproteins can promote certain single steps, such as only adhesion, receptor binding or fusion with the endosomal membrane. An example of a viral glycoprotein that does it all is the spike (S) protein of coronaviruses, an example of two viral glycoproteins that are needed for infection is measles virus with its F and H glycoproteins, and herpesviruses use up to 5 or more virally encoded proteins that are necessary for individual steps in the infection process or need to form hetero-complexes to reach functionality. Expression of viral glycoproteins in any cell will change the protein composition of EVs, which the cell releases. Glycoproteins of enveloped viruses end up on the membranes of EVs, such that the EVs now have acquired new functions. If a single glycoprotein of a virus with an extremely broad cell tropism is used, such EVs can "infect" many different cells. In short, EVs are produced by transient transfection of cells (HEK293 cells, for example) with expression plasmids encoding a viral glycoprotein (spike of SARS-CoV-2, for example) and are able to contain a label as defined herein below. EVs may be directly used from cell culture supernatants or can be purified by biochemical means.

In general, enveloped viruses use the EV export route of their host cells in which they replicate. EVs from such virus-producing cells differ from normal EVs, because their envelope membranes then contain viral glycoproteins in addition to proteins that are present in all EVs, often in abundant amounts. The lumen of these EVs from virus-producing cells then also contains (among other viral factors) the viral genome, which is either bound to a viral protein ("nuclear" capsid protein in SARS-CoV-2) or is contained in a hollow body (= capsid) whose walls also consist of viral proteins. Proteins that are incorporated into the membrane or the envelope of EVs are known, e.g. CD63, CD37, CD53, CD81, CD82, CD54 (ICAM1), CD9, CD151, TSPAN-8 (tetraspanins), integrins, such as alpha-3, -5, -V, -6 and beta-1 and -3, or type I membrane proteins, however, any known protein contained in EVs can therefore be used. A special type of proteins are those that are preferably sorted into EV membranes such as CD63. CD63 is an abundant membrane protein in EVs. Consequently, CD63 promotes the incorporation of proteins or protein domains with which it is covalently fused into the membrane of EVs. The enzymatic activity is now contained in the lumen of the EVs.

The extracellular vesicles used in the method of the present invention may contain viral, receptor-targeting and fusogenic glycoproteins and may be detectable.

Adsorption of a viral particle to a target cell and its penetration into the cytoplasm are the first steps of the viral replication cycle. Membrane-enveloped viruses such as retroviruses use viral glycoproteins embedded into the lipid membrane of their viral particle for these processes. The "viral glycoproteins", as used herein and in the context of the present invention, may comprise proteins that mediate binding to specific cellular receptors on the surface of target cells and/or catalyze the fusion of viral and cellular lipid membrane to allow the release of the viral capsid into the cytoplasm of the target cell so that subsequent steps of the viral replication cycle can start in the cytoplasm or inside the nucleus of the virus-infected cell depending on the virus species. The "viral glycoproteins", as used herein and in the context of the present invention, may be the viral protein being responsible for entry into a/ the cell. Retroviral glycoproteins are usually translated as a precursor protein encoded by the envelope *(env)* open reading frame (ORF). Cotranslational targeting of the glycoproteins to the secretory pathway is mediated by an N-terminal signal sequence, which generally is co-translationally removed. During its transport to the cell surface, the precursor glycoprotein can be further processed by cellular proteases into surface (SU) and transmembrane (TM) subunits. The SU subunit is attached to the extracellular domains of the TM subunit through covalent or non-covalent interactions. Most retroviral glycoproteins form trimeric complexes of the SU/TM heterodimer at the viral or cellular membrane. In general, the SU subunit contains the receptor binding domain (RBD) for the specific cellular receptor used for entry, whereas the fusion machinery utilized for fusion of viral and cellular lipid membranes during virus entry is part of the TM subunit. The TM subunit usually has an exogenous part that mediates the fusion. One specific example is SARS-CoV-2, wherein a S1 subunit makes the receptor contact, and wherein a S2 subunit, which has the TM domain and is anchored with the TM domain in the virus envelope, mediates the fusion.

In the context of the present invention, the term "label" may comprise a protein, which enables the detection of an extracellular vesicle as defined herein. Such a protein may be, for example, a reporter protein, wherein the possibilities how this reporter protein is attached to the extracellular vesicle or components thereof are specified in more details herein below. By the method of the present invention, it is prevented that the label is released from the cells in a soluble form, such that the label is present in the EV preparation in a free form or can be taken up by cells irrespective of a viral glycoprotein. This is the case with enzymes such as luciferases if they are not fused to other proteins that mediate the localization and compartmentalization of the enzyme into EVs. The label is preferably ß-lactamase, however, can be replaced by other enzymes. One further example is ß-galactosidase, wherein its readout is even more compatible with established high throughput technologies, compared to ß-lactamase.

In the context of the present invention, the term "detectable" or "detectable via" means to discover, to discern or to ascertain the existence or the presence of something, in the context of the present invention, the existence or the presence of virus-neutralizing antibodies as defined herein.

As used herein, the term "taking up" means any process to absorb or to incorporate something, e.g., in the context of the present invention, the process leading thereto that the extracellular vesicles are incorporated into said cells as defined herein. "Capable of taking up" instead means that said cell is in principle able to perform such a process to absorb or to incorporate the respective extracellular vesicles.

As used herein, in the context of the present invention, the term "reduced" or "reduced uptake" means that, compared to the control as defined herein, the respective value under investigation has decreased. In the context of the method of the present invention, this means that the value measured with regard to the uptake of extracellular vesicles into said cells, if said cells, said sample and said extracellular vesicles are contacted, is lower, decreased or reduced in comparison to the control, wherein said cells and said extracellular vesicles are contacted, but without said sample.

In the context of the present invention, the term "control" or "in comparison to a control" means comparing the state, wherein said cells, said sample and said extracellular vesicles are contacted, with the state, wherein said cells and said extracellular vesicles are contacted, but without said sample, with regard to the uptake of EVs into said cells, and thus also with regard to the presence or absence of virus-neutralizing antibodies.

As used herein, in the context of the present invention, the term "contacted" means to put or bring something into contact with something, e.g. in the context of the present invention, the sample to be investigated with respect to the presence of virus-neutralizing antibodies with said extracellular vesicles and said cells.

In the context of the present invention, the term "is indicative of" or "is indicative of the presence/ absence" means a process serving to indicate something, e.g. in the context of the present invention, that the presence of virus-neutralizing antibodies is given or not given.

The method for determining whether or not virus-neutralizing antibodies are present in a sample obtained from a subject, can focus thereon if a fusion of the extracellular vesicles with said cells is present and does not require any de novo gene expression of components, e.g. the expression of a full set of viral proteins. Consequently, the method according to the present invention is fast and can be completed within a few hours. The needed materials are not infectious or contagious and can be handled in standard laboratories. Moreover, the method according to the present invention does not require a permission to work with infectious pathogens as the EVs contain no genetic information and cannot propagate infection. The methods of the present invention also provide high-throughput options and can be highly automated.

It is further described herein (not according to the invention) that the method for determining whether or not virus-neutralizing antibodies are present in a sample obtained from a subject, comprises the following steps:
- providing extracellular vesicles and a label, wherein the extracellular vesicles are non-infectious, comprise one or more viral glycoprotein(s) and are detectable via the label,
- contacting the sample with said extracellular vesicles and cells, which are capable of taking up said extracellular vesicles, wherein the one or more viral glycoprotein(s) is/ are able to target a receptor of said cells and is/ are fusogenic, and
wherein no change in the uptake of said extracellular vesicles by said cells in comparison to a control, wherein said cells and said extracellular vesicles are contacted, but without said sample, is indicative of the absence of virus-neutralizing antibodies.

As used herein, the term "no change in the uptake of said extracellular vesicles" means that, compared to the control as defined herein, the respective value under investigation has stayed the same. In the context of said method, "no change in the uptake of said extracellular vesicles" means that the value measured with regard to the uptake of extracellular vesicles into said cells, if said cells, said sample and said extracellular vesicles are contacted, is about the same or has stayed the same in comparison to the control, wherein said cells and said extracellular vesicles are contacted, but without said sample. This condition is then indicative of the absence of virus-neutralizing antibodies.

It is preferred for the method of the present invention that the label is a protein selected from the group consisting of beta-galactosidase, beta-lactamase, beta-glucuronidase, a luciferase and any combination thereof. The luciferase may be a firefly luciferase or a renilla luciferase. For example, the luciferase may also be a luciferase selected from the group consisting of the North American firefly luciferase, e.g. from the organism *Photinus pyralis,* the Japanese firefly luciferase, e.g. from the organism *Luciola cruciate* or the organism *Luciola lateralis,* the Italian firefly luciferase, e.g. from the organism *Luciola italic,* the East European firefly luciferase, e.g. from the organism *Luciola mingrelica,* the Pennsylvania firefly luciferase, e.g. from the organism *Photuris pennsylvanica,* the click beetle luciferase, e.g. from the organism *Pyrophorus plagiophthalamus,* the Railroad worm luciferase, e.g. from the organism *Phrixothrix hirtus,* which all can use D-luciferin as a substrate. The luciferase may also be *Renilla* luciferase, Rluc8 (mutant of *Renilla* luciferase) or Green *Renilla* luciferase, e.g. from the organism *Renilla reniformis, Gaussia* luciferase or Gaussia-Dura luciferase, e.g. from the organism *Gaussia princeps, Metridia* luciferase, e.g. from the organism *Metridia longa,* or OLuc, e.g. from the organism *Oplophorus gracilorostris,* which all can use coelenterazine as substrate. A luciferase, which may be used in the context of the present invention may also be the *Cypridina* luciferase, e.g. from the organism *Cypridina noctiluca* or the organism *Cypridina hilgendorfii,* which can use *vargulin*/ cypridinaluciferin as substrate. The term "luciferase" as used herein may comprise an artificial or partially synthetic or engineered luciferase, such as Nanoluciferase from Promega, for example.

Beta-galactosidase, also called lactase, beta-gal or β-gal, is a glycoside hydrolase enzyme that catalyzes the hydrolysis of β-galactosides into monosaccharides through the breaking of a glycosidic bond. For example, the beta-galactosidase activity or label may be detected via a fluorescent probe or fluorescent molecule, e.g. fluorescein di-β-D-galactopyranoside (FDG) or a β-galactosidase activity detecting probe, such as GlycoGreen-β-Gal. E.g., the latter is a fluorescent probe to detect β-galactosidase activity. It is a non-fluorescent substrate for β-galactosidase enzyme and fluoresces upon the reaction with the enzyme. It is a cell permeable reagent and after the reaction, the generated fluorophore associates with intracellular structures. Low cytotoxicity of this reagent enables live cell imaging without interfering cellular functions. Further, the Galacto-Star^{™} One-Step β-galactosidase Reporter Gene Assay system may be used as a β-galactosidase activity detecting probe, which is a chemiluminescent reporter gene assay system, enabling sensitive detection of β-galactosidase.

Beta-glucuronidase is a member of the glycosidase family of enzymes that catalyze breakdown of complex carbohydrates. Human β-glucuronidase is a type of glucuronidase (a member of glycosidase Family 2) that catalyzes hydrolysis of β-D-glucuronic acid residues from the non-reducing end of mucopolysaccharides (also referred to as glycosaminoglycans) such as heparan sulfate. For the present invention,the beta-glucuronidase activity or label may be detected via a fluorescent probe or fluorescent molecule, e.g. TokyoGreen-β-GIcU(Na).

Beta-lactamases are enzymes produced by bacteria that provide multi-resistance to β-lactam antibiotics, such as penicillins, cephalosporins, cephamycins, and carbapenems (ertapenem), although carbapenems are relatively resistant to beta-lactamase. In the present invention, the beta-lactamase activity or label may be detected via a fluorescent probe or fluorescent molecule, which are well known to a person skilled in the art. CCF4 or CCF2 may be used as such a fluorescent probe or fluorescent molecule, e.g. of these two CCF4 and e.g. an lipophilic, esterified form of CCF4, CCF4-AM. CCF4 is a Fluorescence Resonance Energy Transfer (FRET) substrate, which consists of a cephalosporin core, linking 7-hydroxycoumarin to fluorescein. It is possible for CCF4-AM to readily enter cells. Upon entry, the cleavage by endogenous cytoplasmic esterases rapidly converts CCF4-AM into its negatively charged form, CCF4, which is retained in the cytosol. The further detection of the fluorescent probe or fluorescent molecule may be done with flow cytometry.

It is preferred for the present invention that the label is a split protein, for example a split protein of beta-galactosidase or a split protein of nanoluciferase.

In one embodiment of the methods of the present invention, the label is a split protein, wherein a first part of the split protein is comprised in the extracellular vesicles and a second part of the split protein is comprised in the cells, and wherein the first and the second part of the split protein are able to form a complex. It is preferred for this embodiment, that the first part of the split protein, which is comprised in the extracellular vesicles, is attached to: (i) an extracellular vesicle protein of the extracellular vesicles as defined herein, or (ii) a viral capsid-protein or a viral nucleo-protein of the extracellular vesicles as defined herein, wherein the one or more viral glycoprotein(s) and the viral capsid-protein or the viral nucleo-protein may be from the same virus; or (iii) a viral tegument protein of the extracellular vesicles, and wherein the one or more viral glycoprotein(s) and the viral tegument protein may be from the same virus, or (iv) the one or more viral glycoprotein(s) of the extracellular vesicles as defined herein. The first part of the split protein may be attached to a protein of the extracellular vesicles selected from the group consisting of a tetraspanin; e.g. CD63, CD40, CD81, CD9, CD37, CD53, CD54, CD151, CD82 and TSPAN-8; an integrin; e.g. alpha-3, alpha-5, alpha-V, alpha-6, beta-1 or beta-3 integrin; and a type I membrane protein. Such proteins that are incorporated into the membrane or the envelope of EVs, however, any known cellular membrane protein contained in EVs can be used to fuse each of them with the first part of the split domain/ protein of the label as described herein. For this embodiment, it may be that the second part of the split protein, which is comprised in the cells, is attached to a protein of the cell selected from the group consisting of a tetraspanin; e.g. CD63, CD40, CD81, CD9, CD37, CD53, CD54, CD151, CD82 and TSPAN-8; an integrin; e.g. alpha-3, alpha-5, alpha-V, alpha-6, beta-1 or beta-3 integrin; SNX3, a Pleckstrin domain, an N-myristoylation domain and a type I membrane protein. Proteins that are incorporated into the plasma membrane or other membranes of cells, e.g. CD63, CD37, CD53, CD81, CD82, CD54 (ICAM1), CD9, CD151, TSPAN-8 (tetraspanins), integrins, such as alpha-3, -5, -V, -6 and beta-1 and -3, or type I membrane proteins, however, any known cellular membrane protein contained in the cells can be used to fuse each of them with the second part of the split domain/ protein of the label as described herein. Additionally, those proteins may be used here to (i) prevent the leakage of the first and/ or second part of the split protein and to (ii) anchor the second part of the split protein in cellular membranes with which the membrane of the incoming extracellular vesicles fuses. For the claimed invention, CD63 is a membrane protein present in the extracellular vesicles. Embodiments without CD63 are disclosed but not claimed.

When a complex between the first part of the split protein and the second part of the split protein is formed, this may mean that the encounter of both parts results in the formation of a state, which is able to assemble or reconstitute the functionally active label, which may be an enzyme or protein. This also means, that the first and the second part of the split protein of the label in such a complex remain two parts and do not join or fuse, even when being present in the same compartment or location, e.g. the cytoplasm of a cell for example. Thus, such a complex may be an active enzymatic complex, e.g. such that upon complex-formation, the first and the second part of the split protein assemble into an active enzymatic protein complex. The term "form" as used herein in the context of the present invention and when used in "to form a complex" can be used synonymously with "constitute", "result in", "assemble" or "lead to the formation of".

The extracellular vesicles as described herein may need to contain minimally a split label (for example a CD63 fusion with carboxyterminal HiBiT label of a nanoluciferase) and e.g. Spike (as an example of a viral glycoprotein).

For example, the inventors of the present invention expressed the second part of the split protein of the label fused to the carboxyterminal ends of CD63, to SNX3, to Pleckstrin, and to the N-myristoylation domain of HIV-gag in recipient cells and/ or recipient extracellular vesicles. The human SNX3 gene encodes Sorting nexin-3, which contains a phox (PX) domain, a phosphoinositide binding domain that targets the protein to the inner leaf of endosomal membranes. Pleckstrin is the equivalent of the Pleckstrin homology domain (PH domain), a protein domain of 120 amino acids that is present in a range of different proteins. The domain is involved in intracellular signaling or as constituents of the cytoskeleton and binds phosphatidylinositol lipids within biological membranes as well as different proteins (βγ-subunits of heterotrimeric G proteins, protein kinase C). Pleckstrin domains recruit proteins to different types of cellular membranes, thus targeting proteins with pleckstrin domains to appropriate cellular compartments. The N-myristoylation domain is a very small protein domain, which is the target of N-myristoylation, i.e., the attachment of a 14-carbon fatty acid, myristate, onto the N-terminal glycine residue of target proteins, catalyzed by N-myristoyltransferase (NMT) contained in eukaryotic cells. Myristoylation promotes membrane binding to ensure stable protein association with cellular membranes. The inventors used the domain derived from HIV-gag, in which the N-terminal glycine is the acceptor for myristoylation, but many other viral and cellular N-myristoylation domains are documented. By this, the inventors aimed at attaching the split protein of the label to inner cellular membranes of the VLP-recipient or target cell to ensure that the label does not leak, i.e., is not secreted into the cells' supernatant. A second advantage is that almost all enveloped viruses use the endosomal pathway to release their viral genetic information (contained in a viral capsid or embedded into a viral ribonucleoprotein complex) into the cytoplasm of the infected cell. The endosomal membrane fuses with the membrane of the enveloped virus such that the split protein of the label (e.g. in the extracellular vesicles) can make immediate contact with the split protein of the label attached to the inner leaf of or incorporated in the endosomal membrane in the recipient cell.

It may be that artificial proteins consisting of the so-called large-bit domain of nanoluciferase (LgBiT; 18 kDa) fused to either CD63, SNX3, Pleckstrin, or the N-myristoylation domain may be used in the methods of the present invention. An N-myristoylation domain for creating the split protein fusion of the label may be used. The split label technology from Promega may also be used in the methods of the present invention as described herein.

Other split label technologies can be easily adapted to other labels, such as ß-galactosidase, and can also be used in the methods of the present invention as described herein.

It is preferred for the method for determining whether or not virus-neutralizing antibodies are present in a sample obtained from a subject of the present invention that the receptor of the cell is selected from the group consisting of angiogenin converting enzyme 2 (ACE2), CD46, CD150 (signaling lymphozyte-activation molecule SLAM), lysosomal associated membrane protein 1 (LAMP1), T-cell immunoglobin mucin domain-1 (TIM-1), sialyl-(alpha-2,3)-galactosidase-receptor, sialyl-(alpha-2,6)-galactosidase-receptor, CD21 and a MHC class II receptor.

In the method for determining whether or not virus-neutralizing antibodies are present in a sample obtained from a subject of the present invention, the one or more viral glycoprotein(s) may be fusogenic. "Fusogenic" may mean in this connection that something is able to perform or take part in a fusion process, especially, relating to cells. Viral glycoproteins, which are incorporated into the viral envelope, ergo EV membrane, target the virus to specific cells (this is the so-called cell tropism of the virus) and, once absorbed into a cell, have such a fusogenic effect (leading to the actual infection process, the unloading of the viral genetic function in the cytoplasm of the targeted cells) to avoid degradation of the infecting virus in endosomal/ lysosomal vesicles. The term "target" may comprise the binding of the one or more viral glycoprotein(s) of the virus to the receptor of a cell.

It is preferred for the method for determining whether or not virus-neutralizing antibodies are present in a sample obtained from a subject of the present invention that the extracellular vesicles are detectable via the label, wherein the label is attached to an extracellular vesicle protein comprised in the extracellular vesicles. It is especially preferred for this embodiment, that the extracellular vesicles comprise the extracellular vesicle protein and the label as a fusion protein. Thus, in this embodiment, said label is attached to a protein contained in the EVs, e.g. beta-galactosidase or beta-lactamase as label, e.g. in the form of a fusion protein. As the protein is inevitably incorporated into the EVs, it is ensured that EVs contain the label, leading to quality assurance of the method of the present invention. Tscherne *et al.* had to use a protease to degrade any leaked fusion proteins. The inventors of the present invention instead did not encounter this problem of protein leakage even when the label is attached to the viral nucleoprotein N of SARS-CoV-2. The inventors of the present invention have further found out that said leakage problem can be controlled, when the label is attached to an extracellular vesicle protein of the EVs, e.g a membrane protein of EVs namely CD63, or when the label is attached to one or more viral glycoprotein(s). Thereby problems can be avoided that might arise from using viral, proteinous components that EV-producing cells secrete for unknown reasons. Attaching/ fusing the label with membrane proteins is preferred here, because cellular membrane proteins namely CD63 or all viral glycoproteins with transmembrane domains do not leak, but are caught, fixed and embedded into cellular membranes, the membranes of EVs or the membranes of enveloped viruses via the transmembrane domain(s), which is advantageous here.

Thus, in one embodiment of the method for determining whether or not virus-neutralizing antibodies are present in a sample obtained from a subject, the extracellular vesicle protein may be selected from the group consisting of a tetraspanin; preferably CD63, CD40, CD81, CD9, CD37, CD53, CD54, CD151, CD82 and TSPAN-8; an integrin; preferably alpha-3, alpha-5, alpha-V, alpha-6, beta-1 or beta-3 integrin; and a type I membrane protein. For the claimed invention, CD63 is a membrane protein present in the extracellular vesicles. Embodiments without CD63 are disclosed but not claimed.

The CD63 antigen is a protein that, in humans, is encoded by the CD63 gene. Said protein is a member of the transmembrane 4 superfamily, also known as the tetraspanin family. Cluster of differentiation 40, also called CD40, is a costimulatory protein found on antigen-presenting cells and is required for their activation. Most of these members are cell-surface proteins that are characterized by the presence of four hydrophobic domains. CD81 molecule, also known as CD81 (Cluster of Differentiation 81), is a protein which in humans is encoded by the CD81 gene. It is also known as 26 kDa cell surface protein, TAPA-1 (Target of the Antiproliferative Antibody 1), and Tetraspanin-28 (Tspan-28). CD9 is also a protein that is a member of the transmembrane 4 superfamily. It is a cell surface glycoprotein that consists of four transmembrane regions and has two extracellular loops that contain disulfide bonds, which are conserved throughout the tetraspanin family. CD37 is also called Tspan-26. CD53 is a leukocyte surface antigen and also a member of the transmembrane 4 superfamily (transpanin family). CD54 (Cluster of Differentiation 54) is also called ICAM-1 (Intercellular Adhesion Molecule 1) and a protein, which is a cell surface glycoprotein, being typically expressed on endothelial cells and cells of the immune system. It binds to integrins of type CD11a/ CD18 or CD11b/ CD18. CD151 (Cluster of Differentiation 151) is a cell surface glycoprotein that is known to complex with integrins and other transmembrane 4 superfamily proteins. CD82 (Cluster of Differentiation 82) is a membrane glycoprotein, which is also called *KAI1.* TSPAN-8, a member of the transmembrane 4 superfamily, is also called CO-029, TM4SF3 or tetraspanin 8. Integrins are transmembrane receptors that facilitate cell-extracellular matrix (ECM) adhesion. Type I (trans)membrane proteins are anchored to the lipid membrane with a stop-transfer anchor sequence and have their N-terminal domains targeted to the endoplasmic reticulum (ER) lumen during synthesis (and the extracellular space, if mature forms are located on cell membranes).

In one embodiment of the method for determining whether or not virus-neutralizing antibodies are present in a sample obtained from a subject of the present invention, the extracellular vesicle protein may be a viral extracellular vesicle protein. Such a viral extracellular vesicle protein may be selected from the group consisting of a corona virus protein; e.g. the E-, M-, S- or N-protein of a SARS-CoV-virus, specifically the E-, M-, S- or N-protein of the SARS-CoV-2-virus; an Epstein-Barr-virus-protein, a measles virus protein, an influenza virus protein, a parainfluenza virus protein, a human respiratory syncytial virus protein, an Ebola virus protein, a hanta virus protein, a Lassa virus protein, and any truncated form thereof. Thus, this embodiment may also include viral extracellular vesicle proteins, which after ectopic expression in suitable cells also become the "extracelular vesicle protein", e.g. land in or on the EVs. The reason for this sorting of viral proteins in or on EVs lies in the (viral) molecular mechanisms that dominate the cellular processes during morphogenesis and egress of enveloped viruses or at least hijack cellular processes of EV biogenesis and convert them for viral purposes. The invention as claimed involves one or more viral glycoproteins. Embodiments lacking this feature are disclosed but not claimed.

Coronaviruses are enveloped RNA viruses and form virions with a diameter of 80-140 nm. They have a single-stranded RNA genome of positive polarity of about 30 kilobases in length, the largest known genome of all RNA viruses. It encodes non-structural proteins responsible for RNA replication, as well as the four structural proteins S, E, M and N. The S, E and M proteins are stored in the viral membrane that envelops the nucleocapsid, which is composed of N protein (nucleo-protein) and the viral genomic RNA molecule. The S (Spike) protein is responsible for entering the host cell and consists of two subunits: the S1 subunit contains the receptor binding domain (RBD), which binds to the host cell receptor; the S2 subunit then mediates the fusion of the viral envelope and cell membrane. The Spike protein induces neutralizing (protective) antibodies and is therefore of the highest interest for vaccine development.

Similarly, viral extracellular vesicle proteins of the Epstein-Barr virus, the measles virus, the influenza virus, the parainfluenza virus, the human respiratory syncytial virus, the Ebola virus, the hanta virus, the Lassa virus are well known to the person skilled in the art. Examples for viral extracellular vesicle proteins are gp350 for Epstein-Barr virus, fusion (F) glycoprotein (also termed Fgp) for measles virus, neuraminidase (NA) for influenza virus, fusion (F) glycoprotein for parainfluenza virus, G lipoprotein for respiratory syncytial virus, glycoprotein GP for Ebola virus, Gc envelope glycoprotein for hanta virus, GP2 glycoprotein for Lassa virus, or Spike protein for SARS-CoV virus. For example, the viral extracellular vesicle protein of the Epstein-Barr virus may be gp350. The viral extracellular vesicle protein of the measles virus may be the fusion (F) glycoprotein. The viral extracellular vesicle protein of the influenza virus may be neuraminidase (NA). The viral extracellular vesicle protein of the parainfluenza virus may be the fusion (F) glycoprotein. The viral extracellular vesicle protein of the respiratory syncytial virus may be the G lipoprotein. The viral extracellular vesicle protein of the Ebola virus may be the glycoprotein GP. The viral extracellular vesicle protein of the hanta virus may be the Gc envelope glycoprotein. Further, the viral extracellular vesicle protein of the Lassa virus is GP2 glycoprotein. The viral extracellular vesicle protein of the SARS-CoV virus may be the Spike protein.

In a further embodiment of the method for determining whether or not virus-neutralizing antibodies are present in a sample obtained from a subject of the present invention, the extracellular vesicles are detectable via the label, wherein the label is attached to a viral capsid-protein or viral nucleo-protein and wherein the one or more viral glycoprotein(s) and the viral capsid-protein or viral nucleo-protein are from the same virus. Within this embodiment, the viral capsid-protein may be selected from the group consisting of HIV-1 and a viral capsid-protein of a herpes virus. It is further preferred for this embodiment that the viral nucleo-protein is selected from the group consisting of a nucleo-protein from an Epstein-Barr virus, a measles virus, an influenza virus, a parainfluenza virus, a human respiratory syncytial virus, an Ebola virus, a Marburg virus, a hanta virus, a Lassa virus and the nucleo-protein N of a SARS-CoV-virus, preferably the nucleo-protein N of the SARS-CoV-2-virus. The viral capsid-protein or the viral nucleo-protein may come from the same virus whose glycoproteins are contained in the EVs. Some RNA viruses do not have a capsid, but proteins, in which the genome of the virus is embedded or attached to. Such proteins are called nucleo-proteins.

In one embodiment of the method for determining whether or not virus-neutralizing antibodies are present in a sample obtained from a subject of the present invention, the extracellular vesicles are detectable via the label, wherein the label is attached to the one or more viral glycoprotein(s) of the extracellular vesicles. For this embodiment, it is preferred that the one or more viral glycoprotein(s) is/ are not the M-, E- or S-protein of the SARS-CoV-2-virus. For this embodiment, it is preferred, for example, that the glycoprotein gp350 of EBV is attached to ß-lactamase as label. For example, the one or more viral glycoprotein(s) may be the S-protein of a SARS-CoV-virus, the N-protein of a SARS-CoV-virus or gp350 of Epstein-Barr virus.

In a further embodiment of the method for determining whether or not virus-neutralizing antibodies are present in a sample obtained from a subject of the present invention, the virus-neutralizing antibodies are neutralizing antibodies againts a virus selected from the group consisting of a coronavirus; for example a SARS-CoV-virus, specifically the SARS-CoV-2-virus; Epstein-Barr virus, measles virus, influenza virus, parainfluenza virus, human respiratory syncytial virus, Ebola virus, hanta virus and Lassa virus.

In one embodiment of the method of the present invention, the one or more viral glycoprotein(s) is/ are on the surface of the extracellular vesicles.

It is further preferred for the method for determining whether or not virus-neutralizing antibodies are present in a sample obtained from a subject of the present invention, that the one or more viral glycoprotein(s) is/ are one or more viral glycoprotein(s) from a virus selected from the group consisting of a coronavirus; for example a SARS-CoV-virus, specifically the SARS-CoV-2-virus; an Epstein-Barr virus, measles virus, influenza virus, parainfluenza virus, human respiratory syncytial virus, Ebola virus, hanta virus and Lassa virus.

It is described herein that in the method for determining whether or not virus-neutralizing antibodies are present in a sample obtained from a subject of the present invention, the one or more viral glycoprotein(s) may be selected from the group consisting of haemagglutinin (HA), neuraminidase (NA), haemagglutinin-neuraminidase (HN), fusion (F) glycoprotein, glycoprotein polyprotein (GP) complex, Ebola virus glycoprotein, glycoprotein pg350 of Epstein-Barr virus, gB (BALF4) of Epstein-Barr virus, BILF2 of Epstein-Barr virus, gp42 (BZLF2) of Epstein-Barr virus, gH (BXLF2) of Epstein-Barr virus, gL (BKRF2) of Epstein-Barr virus, glycoprotein gp120 of HIV, M-, E- and S-protein of the SARS-CoV-virus.

In case of SARS-CoV-2, the Spike (S)-protein targets ACE2 (Angiotensin Converting Enzyme 2) in combination with priming by TMPRSS2, also called transmembrane protease serin 2, a cellular protease.

With regard to measles virus, the two cell surface receptors, CD46 and signaling lymphocyte-activation molecule SLAM (CD150), depending on the measle strain, have been identified and interact with the viral glycoprotein haemagglutinin (HA, also termed Hgp). Measle virus fusion (F) glycoprotein, Fgp, mediates membrane fusion afterwards.

Both A and B influenza viruses contain two major surface glycoproteins, the haemagglutinin (HA), possessing the receptor-binding and fusion activities, and the neuraminidase (NA), which destroys the receptor by cleaving sialic acid from host cell membranes, thereby releasing newly formed virus particles into the cell's cytoplasm. HA binds to and uses sialic acid-containing molecules as also described herein as receptors.

Lassa virus entry is a two-step process involving the unique trimeric viral glycoprotein polyprotein (GP) complex, viral uptake into endosomes and the following fusion with the endosomal membrane, all mediated by subcomponents of GP. The cellular receptor is LAMP1 (lysosomal associated membrane protein 1).

Concerning Ebola virus, Ebola virus glycoprotein (GP) and its differently spliced and frame-shifted versions from a single gene may be used as the one or more viral glycoprotein(s), wherein the glycoprotein interacts with T cell immunoglobulin mucin domain-1 (TIM-1), a phosphatidylserine (PS) receptor. The same applies for the Marburg virus.

For parainfluenza viruses, the receptor-binding glycoprotein haemagglutinin-neuraminidase (HN) and the fusion (F) glycoprotein may be used as the one or more viral glycoprotein(s). The sialyl-(α-2,3)-galactosidase receptor is relevant for human parainfluenza virus type 1 and avian influenza viruses, while for human influenza viruses it is the sialyl-(α-2,6)-galactosidase receptor.

For the Epstein-Barr virus, several glycoproteins, such as gp350 (BLLF1), gB (BALF4), BILF2, gp42 (BZLF2), gH (BXLF2) or gL (BKRF2) may be used as the one or more viral glycoprotein(s). Known cellular receptors are CD21 and MHC class II receptors, being well known to a person skilled in the art.

In one further embodiment of the method for determining whether or not virus-neutralizing antibodies are present in a sample obtained from a subject of the present invention, the extracellular vesicles are detectable via the label, wherein the label is attached to a viral tegument protein of the extracellular vesicles, and wherein the one or more viral glycoprotein(s) and the viral tegument protein are from the same virus. A viral tegument or tegument, more commonly known as a viral matrix, is a cluster of proteins that lines the space between the envelope and nucleo-capsid of e.g. all herpesviruses. The tegument generally contains proteins that aid in viral DNA replication and evasion of the immune response, typically inhibiting signaling of the immune system and activation of interferons. The tegument is usually released shortly after infection into the cytoplasm. These proteins are usually formed within the late phase of the viral infectious cycle, after viral genes have been replicated. Viral teguments can be symmetrically arranged via structural and scaffolding protein or can also be asymmetrically arranged, depending on the virus. Teguments usually involve scaffolding proteins in their formation around the nucleo-capsid. Non-essential proteins included in the tegument may aid in immune response suppression, suppression of host mRNA transcription or suppression of intrinsic or cellular defenses. Essential proteins will include factors that help in trafficking of the viral capsid to the nucleus (for herpesviruses), recruiting host transcription or translation factors, or directly transcribing or translating viral genes. Tegumental contents are released into the cytoplasm upon entrance into the cell upon which many tegumental proteins become active. The tegument may also aid in insertion of the viral genome into host cell cytoplasm or nucleus.

Thus, the detectability of extracellular vesicles is mediated by a label attached to either (i) an EV-protein or (ii) a viral capsid-protein or a viral nucleo-protein or (iii) a viral glycoprotein or (iv) a viral tegument protein. In each of these cases, it is ensured that the label will be contained in the EVs. E.g. with regard to (i), the label will definitely be included in the EV, because it is attached to an EV-protein. The viral glycoprotein ensures that the EV (with label) binds to said cell. By this way, the EV-protein targets the label into the EV and the viral glycoprotein targets the EV (now equipped with a label) to and into said cell. In case (ii), the EVs are functional in that the viral glycoproteins ensure that the viral capsid protein or viral nucleo-protein (with label) from the same virus will be in the EV and the EV in turn contains all the components that target it to said cell and it is in turn detectable. This provides the advantage that the one or more viral glycoprotein(s) and the viral capsid protein or viral nucleo-protein will certainly be present in the EV, because the viral glycoproteins ensure that the viral capsid protein or viral nucleo-protein is assembled together with them. In case (iii), the EVs are functional in that the viral glycoproteins (with label) are in the EV, which in turn contains all the components that are necessary to make the viral glycoproteins and the viral capsid protein/ nucleo-protein function. The same applies for case (iv). Additionally, the present invention also comprises that in one embodiment, the label is a split protein, wherein a first part of the split protein is comprised in the extracellular vesicles and a second part of the split protein is comprised in the cells, and wherein the first and the second part of the split protein are able to form a complex, e.g. such that the first and the second part of the split protein are able to assemble into the functionally active label.

According to the method of the present invention, the extracellular vesicles are non-infectious. The term "non-infectious" as used in the context of the present invention means that the vesicle(s) fuse(s) or is/ are able to fuse with a target cell, but the vesicle(s) does/ do not propagate viral infection.

In one further embodiment of the method of the present invention, said cell is a cell selected from the group consisting of a primary cell, a cell line, an epithel cell, an immune cell and a cell line engineered to express a viral receptor, preferably a viral receptor selected from the group consisting of angiogenin converting enzyme 2 (ACE2), CD46, CD150 (signaling lymphozyte-activation molecule SLAM), lysosomal associated membrane protein 1 (LAMP1), T-cell immunoglobin mucin domain-1 (TIM-1), sialyl-(alpha-2,3)-galactosidase-receptor, sialyl-(alpha-2,6)-galactosidase-receptor, CD21 and a MHC class II receptor. Cells are preferentially of human origin, but can also be selected from other species. For example, Vero cells may be used. It may be that immune cells are used, such as B-cells, T-cells or macrophages, specifically CD19 positive lymphocytes.

In a further aspect, the present invention further provides a method for determining whether or not virus-neutralizing antibodies are present in a sample obtained from a subject, comprising the following steps:
- providing a donor type of extracellular vesicles and a label,
   wherein the donor type of extracellular vesicles
   (i) are non-infectious,
   (ii) comprise one or more viral glycoprotein(s),
   (iii) are detectable via the label,
   (iv) are free of recombinant genetic material, and
   (v) comprise proteins that are incorporated into the membrane or the envelope of the extracellular vesicles, wherein a membrane protein in the extracellular vesicles is CD63;
- contacting the sample with said donor type of extracellular vesicles and a recipient type of extracellular vesicles, which are capable of taking up said donor type of extracellular vesicles, and
- determining whether or not said recipient type of extracellular vesicles take up said donor type of extracellular vesicles;
   wherein a reduced uptake of said donor type of extracellular vesicles by said recipient type of extracellular vesicles in comparison to a control, wherein said donor type of extracellular vesicles and said recipient type of extracellular vesicles are contacted, but without said sample, is indicative of the presence of virus-neutralizing antibodies.

The term "donor type of extracellular vesicles" as used herein, may mean a type of extracellular vesicles that carry one or more viral glycoprotein(s) and, if present, the first part of the split protein of the label, which should be transferred to the recipient type of extracellular vesicles. The term "recipient type of extracellular vesicles" as used herein, may mean a type of extracellular vesicles that carry the receptor of the respective one or more viral glycoprotein(s) and, if present, the corresponding second part of the split protein of the label and may act as extracellular vesicles, which are targeted by said donor type of extracellular vesicles. The term "type" of extracellular vesicles as used herein and in the context of the present invention can be used interchangeably with "class" of extracellular vesicles. Extracellular vesicles may be classified to belong to the same type or class, e.g. based on their size or content or receptors they comprise.

Thus, one aspect of the present invention uses a recipient type of extracellular vesicles, which - similar to the cells as described herein, which may be recipient or target cells - are capable of taking up the extracellular vesicles/ the donor type of extracellular vesicles, i.e., capable of promoting or supporting the fusion with the donor type of extracellular vesicles. After a fusion between an extracellular vesicle of the donor type and an extracellular vesicle of the recipient type, they then share the same membrane structure and their cargoes have united. Lipid reorganization and protein restructuring can be part of that fusion process.

Both types of extracellular vesicles may be designed to comprise split proteins of a label as described herein. Thus, the present invention also comprises that in one embodiment, the label is a split protein, wherein a first part of the split protein is comprised in the extracellular vesicles, which belong to said donor type of the extracellular vesicles, and a second part of the split protein is comprised in the extracellular vesicles, which belong to said recipient type of extracellular vesicles, and wherein the first and the second part of the split protein are able to form a complex, e.g. such that the first and the second part of the split protein are able to assemble into the functionally active label. Thus in this embodiment, the donor type of extracellular vesicle may be detectable only, if they fuse with the recipient type of extracellular vesicles, which may contain the complementing part of the split protein of the label. As a consequence, both types of extracellular vesicles need to be able in this embodiment to fuse such that the two split proteins of the label can be able to form the complete and active complex of the label. The split proteins of the label may be usually inactive when contained in separate, non-fused extracellular vesicles in a suspension, which may contain high concentrations of both types of extracellular vesicles.

The recipient type of extracellular vesicles, which serve as a recipient for the donor type of extracellular vesicles according to the methods of the present invention, may be produced in and released from cells that express ACE2 (the receptor of Spike) and the complementing protein of the label (e.g. the LgBiT label, as described above, also as a carboxyterminal fusion with e.g. CD63). The inventors of the present invention have found that the two types, the donor and recipient types of extracellular vesicles may fuse such that the split proteins of the label reassemble into the fully active form of the label, e.g. in the lumen of the two types of extracellular vesicles upon their fusion. The fusion may depend on the presence of (i) the one or more viral glycoprotein(s) and (ii) its matching cellular receptor on the donor and recipient type of extracellular vesicles, respectively. Sera that contain neutralizing antibodies directed against the viral glycoprotein or monoclonal antibodies with neutralizing properties may be able to block the fusion of the two types of extracellular vesicles in a dose-dependent fashion as is shown in Figure 6. The advantageous effect in this regard is that the virus-like particle neutralization test can work with cell-free components, which can be directly used without the need to cultivate recipient or target cells. Moreover, the cell-free components, the donor and recipient types of extracellular vesicles can be frozen, stored long-term and used directly after thawing very much in contrast to viably frozen recipient or target cells.

For example, the inventors incubated the donor type of extracellular vesicles as described herein with serum dilutions for a defined period (e.g. 30 min), added the recipient type of extracellular vesicles, and incubated the suspension for e.g. 4 hours at 37°C. To concentrate the extracellular vesicles in the suspension (single as well as fused extracellular vesicles), it is e.g. possible to precipitate and collect them with magnetic beads (with a specificity to bind glycoproteins, for example). By adding the substrate of the label to the magnetic beads, light that emits directly from the bead-bound extracellular vesicles can be measured. This approach provides the advantage that one may need extremely little substrate to analyze the highly concentrated extracellular vesicles, due to being able to discard the suspension, which contained them.

The present invention further provides an *in vitro* method for screening (a) compound(s) for its/ their ability to neutralize a virus, comprising the following steps:
- providing extracellular vesicles and a label,
   wherein the extracellular vesicles
   (i) are non-infectious,
   (ii) comprise one or more viral glycoprotein(s),
   (iii) are detectable via the label,
   (iv) are free of recombinant genetic material, and
   (v) comprise proteins that are incorporated into the membrane or the envelope of the extracellular vesicles, wherein a membrane protein in the extracellular vesicles is CD63;
- contacting said compound(s) with said extracellular vesicles and cells, which are capable of taking up said extracellular vesicles, wherein the one or more viral glycoprotein(s) is/ are able to target a receptor of said cells and is/ are fusogenic, and
- determining whether or not said cells take up said extracellular vesicles;
wherein a reduced uptake of said extracellular vesicles by said cells in comparison to a control, wherein said cells and said extracellular vesicles are contacted, but without said compound(s), is indicative of the ability of said compound(s) to neutralize said virus, i.e. prevent or hinder it to infect or fuse with a viral target cell.

The term "compound" as used herein for the *in vitro* method for screening (a) compound(s) for its/ their ability to neutralize a virus and as used in the context of the present invention, comprises antibodies, sera, e.g from an individual, or any biological probe or sample, e.g. also such a biological sample as defined herein above.

For the *in vitro* method for screening (a) compound(s) for its/ their ability to neutralize a virus of the present invention, the same definitions as for the method of the present invention as given above apply.

It is further described herein for the *in vitro* method for screening (a) compound(s) for its/ their ability to neutralize a virus of the present invention that the label may be a protein selected from the group consisting of beta-galactosidase, beta-lactamase, beta-glucuronidase, a luciferase and any combination thereof.

Additionally, the present invention also comprises that in one embodiment of the *in vitro* method for screening (a) compound(s) for its/ their ability to neutralize a virus, the label is a split protein, wherein a first part of the split protein is comprised in the extracellular vesicles and a second part of the split protein is comprised in the cells, and wherein the first and the second part of the split protein are able to form a complex.

It is further described herein for the *in vitro* method for screening (a) compound(s) for its/ their ability to neutralize a virus of the present invention that the virus is selected from the group consisting of a coronavirus; e.g. a SARS-CoV-virus, specifically the SARS-CoV-2-virus; Epstein-Barr virus, measles virus, influenza virus, parainfluenza virus, human respiratory syncytial virus, Ebola virus, hanta virus and Lassa virus.

It is further described herein for the *in vitro* method for screening (a) compound(s) for its/ their ability to neutralize a virus of the present invention that the one or more viral glycoprotein(s) is/ are one or more viral glycoprotein(s) from a virus selected from the group consisting of a coronavirus; e.g. a SARS-CoV-virus, specifically the SARS-CoV-2-virus; an Epstein-Barr virus, measles virus, influenza virus, parainfluenza virus, human respiratory syncytial virus, Ebola virus, hanta virus and Lassa virus.

It is further described herein for the *in vitro* method for screening (a) compound(s) for its/ their ability to neutralize a virus of the present invention that the one or more viral glycoprotein(s) is/ are selected from the group consisting of haemagglutinin (HA), neuraminidase (NA), haemagglutinin-neuraminidase (HN), fusion (F) glycoprotein, glycoprotein polyprotein (GP) complex, Ebola virus glycoprotein, glycoprotein pg350 of Epstein-Barr virus, gB (BALF4) of Epstein-Barr virus, BILF2 of Epstein-Barr virus, gp42 (BZLF2) of Epstein-Barr virus, gH (BXLF2) of Epstein-Barr virus, gL (BKRF2) of Epstein-Barr virus, glycoprotein gp120 of HIV, M-, E- and S-protein of the SARS-CoV-virus.

The present invention further provides an *in vitro* method for screening (a) compound(s) for its/ their ability to neutralize a virus, comprising the following steps:
- providing a donor type of extracellular vesicles and a label,
   wherein the extracellular vesicles
   (i) are non-infectious,
   (ii) comprise one or more viral glycoprotein(s),
   (iii) are detectable via the label,
   (iv) are free of recombinant genetic material, and
   (v) comprise proteins that are incorporated into the membrane or the envelope of the extracellular vesicles, wherein a membrane protein in the extracellular vesicles is CD63;
- contacting said compound(s) with said donor type of extracellular vesicles and a recipient type of extracellular vesicles, which are capable of taking up said donor type of extracellular vesicles, and
- determining whether or not said recipient type of extracellular vesicles take up said donor type of extracellular vesicles;
   wherein a reduced uptake of said donor type of extracellular vesicles by said recipient type of extracellular vesicles in comparison to a control, wherein said donor type of extracellular vesicles and said recipient type of extracellular vesicles are contacted, but without said compound(s), is indicative of the ability of said compound(s) to neutralize said virus.

For said *in vitro* method for screening (a) compound(s) for its/ their ability to neutralize a virus of the present invention comprising two types of extracellular vesicles, the same definitions as for the methods of the present invention as given above apply.

It is described herein for the *in vitro* method for screening (a) compound(s) for its/ their ability to neutralize a virus comprising two types of extracellular vesicles that the label is a protein selected from the group consisting of beta-galactosidase, beta-lactamase, beta-glucuronidase, a luciferase and any combination thereof.

Additionally, the present invention also comprises that in one embodiment of the *in vitro* method for screening (a) compound(s) for its/ their ability to neutralize a virus comprising two types of extracellular vesicles, the label is a split protein, wherein a first part of the split protein is comprised in the donor type of extracellular vesicles and a second part of the split protein is comprised in the recipient type of extracellular vesicles, and wherein the first and the second part of the split protein are able to form a complex.

It is further described herein for the *in vitro* method for screening (a) compound(s) for its/ their ability to neutralize a virus comprising two types of extracellular vesicles that the virus is selected from the group consisting of a coronavirus; e.g. a SARS-CoV-virus, specifically the SARS-CoV-2-virus; Epstein-Barr virus, measles virus, influenza virus, parainfluenza virus, human respiratory syncytial virus, Ebola virus, hanta virus and Lassa virus.

It is further described herein for the *in vitro* method for screening (a) compound(s) for its/ their ability to neutralize a virus comprising two types of extracellular vesicles that the one or more viral glycoprotein(s) is/ are one or more viral glycoprotein(s) from a virus selected from the group consisting of a coronavirus; e.g. a SARS-CoV-virus, specifically the SARS-CoV-2-virus; an Epstein-Barr virus, measles virus, influenza virus, parainfluenza virus, human respiratory syncytial virus, Ebola virus, hanta virus and Lassa virus.

It is further described herein for the *in vitro* method for screening (a) compound(s) for its/ their ability to neutralize a virus comprising two types of extracellular vesicles, the one or more viral glycoprotein(s) is/ are selected from the group consisting of haemagglutinin (HA), neuraminidase (NA), haemagglutinin-neuraminidase (HN), fusion (F) glycoprotein, glycoprotein polyprotein (GP) complex, Ebola virus glycoprotein, glycoprotein pg350 of Epstein-Barr virus, gB (BALF4) of Epstein-Barr virus, BILF2 of Epstein-Barr virus, gp42 (BZLF2) of Epstein-Barr virus, gH (BXLF2) of Epstein-Barr virus, gL (BKRF2) of Epstein-Barr virus, glycoprotein gp120 of HIV, M-, E- and S-protein of the SARS-CoV-virus.

Unless otherwise specified, the terms used herein have their common general meaning as known in the art.

Unless otherwise indicated, the term "at least" preceding a series of elements is to be understood to refer to every element in the series.

The term "and/or", wherever used herein, includes the meaning of "and", "or" and "all or any other combination of the elements connected by said term".

When used herein, the term "about" is understood to mean that there can be variation in the respective value or range (such as pH, concentration, percentage, molarity, number of amino acids, time etc.) that can be up to 20 %, up to 10 % or up to 5 % of the given value, including the respective value.

The term "less than" or in turn "more than" does not include the concrete number. For example, "less than 20" means less than the number indicated. Similarly, "more than" or "greater than" means more than or greater than the indicated number, e.g. "more than 80 %" means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps, but not the exclusion of any other integer or step or group of integer or step. When used herein the term "comprising" can be substituted with the term "containing" or "including" or sometimes, when used herein, with the term "having". When used herein, "consisting of" excludes any element, step, or ingredient not specified.

The term "including" means "including but not limited to". "Including" and "including but not limited to" are used interchangeably.

A better understanding of the present invention and of its advantages will be had from the following examples.

### EXAMPLES

### Materials and methods:

### Cell lines and cell culture

Different cell lines were used to produce extracellular vesicles (EVs) equipped with different viral proteins. Preferentially, the inventors chose 293T cells as EV producers and U251MG cells as recipients. The cells were kept in D-MEM medium (Life Technologies) supplemented with 10 % FBS (Life Technologies), penicillin (100 U/ml; Life Technologies), and streptomycin (100 mg/ml; Life Technologies). As an alternative, the inventors also used HEK293-based EB-VLP producer cell lines to generate EVs with viral glycoproteins derived from Epstein-Barr virus (EBV) as described elsewhere. These cells were maintained in RPMI 1640 medium (Life Technologies). All media were supplemented with 10 % FBS (Life Technologies), penicillin (100 U/ml; Life Technologies), and streptomycin (100 mg/ml; Life Technologies). All cells were cultivated at 37°C in a 5 % CO₂ incubator. 293T cells and HEK293 cells were obtained from the Leibniz Institut Deutsche Sammlung von Mikroorganismen und Zellkulturen GmbH [DSMZ], Braunschweig, Germany.

### Molecular cloning of expression vector plasmids encoding viral genes

Preferentially, the inventors purchased the protein-coding sequences as synthetic, custom-specific DNA from a commercial supplier. The nucleotide sequences were codon-optimized and elements known to interfere with transcription or translation were eliminated in this customized step. The synthetic DNAs were ordered based on sequence information available in public data bases (i.e. Genbank or Uniprot). The synthetic DNAs were equipped with suitable restriction enzyme cleavage sites to allow their molecular cloning directly into suitable expression plasmids. Among them, vectors of the pcDNA3 family are preferred to express viral proteins transiently in 293T and HEK293 cells.

### Transient transfection of expression plasmids, preparation and storage of the EV-containing supernatants

3x10⁵ cells were seeded in 2 ml cell culture medium in a single well of a 6-well cluster plate. Twenty-four hours later, the cells were transfected by chemical means (below) by simply adding the DNA complex to the cell culture medium. Two days later, the supernatants were harvested and centrifuged at 330 g and 1,000 g for 10 min each. Aliquots of the supernatants were frozen at -20°C, -70°C or stored at 4°C depending on the physical and biological stability of the EVs. The extracellular particles contained in the purified supernatants can be further purified and concentrated as needed.

### Chemical complexation of expression plasmid vector DNA for transfection

Transient transfection of DNA into 293T cells, HEK293 cells or HEK293 cells with EV-VLP genomes can be achieved by any standard technique. The inventors used complexation of the DNA with TranslT^{®}-293 Transfection Reagent by Mirus, following the manufacturer's protocol. Briefly, 1 µg of plasmid DNA was diluted in 200 µl Optimem medium (ThermoFisher Scientific). 3 µl of TranslT-293 transfection reagents was added, the content of the vial was mixed and incubated at room temperature for 15 to 30 min before the complex was directly added to the cells to be transfected.

HEK293 EB-VLP producer cells were seeded with a density of 1.1×10⁵ cells/cm² in fully complemented cell culture medium supplemented with 0.5 to 1.0 µg/ml puromycin for 24 hours as described. Then, the cells were transfected with chemically complexed expression vector plasmid DNAs as described in the previous paragraph. 24 hours later, the medium was replaced with RPMI1640 cell culture medium without supplements, but with 1 µM 4-hydroxy-tamoxifen. Cells were kept for 3 days when the supernatant with the EB-VLPs was collected and processed further as described in [0123].

### Construction of expression plasmids encoding fusion proteins consisting of viral components and a label

Most viral glycoproteins are type I transmembrane proteins as are tetraspanins and integrins present and enriched in the membranes of EVs. The inventors therefore fused the label (e.g. ß-lactamase, ß-galactosidase, nanoluc, etc.) to the carboxy-terminus of these membrane proteins such that the label localizes to the lumen of the EVs. Similarly, the inventors fused the label to viral nucleoproteins and viral tegument proteins. All fusion proteins benefit from a separation of the two protein parts. To achieve this, the inventors fused the two parts employing a stretch of amino acids consisting of one or more repeats of the peptide N-glycin-glycin-glycin-glycin-serine-C, a known unstructured flexible linker protein domain. To establish expression vectors encoding these fusion proteins, the inventors used existing DNA sequences freely available from common plasmids or purchased synthetic DNAs encoding the label similar to the approach described in [0123]. The fusion protein encoding DNA fragments were cloned into common expression vector plasmids, preferentially of the pcDNA3 vector plasmid family. Expression vector plasmids encoding fusion proteins with labels as specified above were transiently co-transfected with viral glycoproteins as described in [0122] to [0126]. Alternatively, the fusion protein encoding DNA fragments were cloned into common retro or lentiviral expression vectors to support the constitutive expression of fusion proteins with labels in cells stably transduced with such retro- or lentiviral vectors, preferentially 293T cells, HEK293 cells, U251MG cells or HEK293 EB-VLP producer cells.

### Example 1:

Modified EVs resemble virus-like particles that can mimic all steps of viral infection. Serial dilutions of (monoclonal) antibodies or serum from COVID-19 patients (Fig. 1) or individuals with latent viral infections (Fig. 2) were incubated with fixed amounts of such EVs (Fig. 2). Antibodies with specificity towards viral glycoproteins present on the EVs' surface bind to them including antibodies with neutralizing functions. Such antibodies prevented "infection" when the EVs were subsequently incubated with suitable target cells/ cells.

Sera from individuals who have never been in contact with a given virus did not contain such neutralizing antibodies (Figures. 1 and 2). The principal set-up method of the present invention relies on viral glycoprotein(s) and a label that is absent in the target cells. It turned out that it is important to provide measures to locate the enzyme to the lumen of EVs, e.g. by fusing the enzyme domain to the carboxy-terminus of type I transmembrane domains, such as CD63 that end up in EVs' membranes.

### Example 2:

Extracellular vesicles used in Figures 3 to 6 were generated by transient transfection of 293T cells with expression plasmids encoding (i) Spike protein of SARS-CoV-2 (Wuhan D614G strain or the B.1.617.2 variant) and (ii) a first part of a split protein of the label fused to the carboxyterminus of CD63. Recipient U251MG cells in Figures 3 to 5 were engineered to express the SARS-CoV-2 receptor ACE2 and a second part of the split protein of the label, consisting of an N-myristoylated split luciferase label. Serial dilutions of the sera were incubated with extracellular vesicles for 30 min, the mixture was transferred to recipient or target cells cultivated in wells of a 96-well cluster plate and incubated for 4 hours. After removal of the supernatant, substrate was added to the recipient U251MG cells, which were measured in a luminometer 2 min after substrate addition.

### Example 3:

In Figure 6, two sera from a naïve and from a vaccinated individual were analyzed in the virus neutralization test using two types of extracellular vesicles in a cell-free setting, namely a "donor type" of extracellular vesicles and a "recipient type" of extracellular vesicles as herein above defined. The donor type of the extracellular vesicles carried the Spike protein of SARS-CoV-2 (Wuhan D614G strain) and a first part of the split protein of the label fused to the carboxyterminus of CD63 as above described in Example 2. The recipient type of extracellular vesicles carried the ACE2 receptor and the corresponding second part of the split protein of the label, which is also fused to CD63. The donor type of extracellular vesicles was harvested from the supernatants of 293T cells that had been transfected with expression plasmids encoding the Spike protein and the first part of the split protein of the label as described above in Example 2. The recipient type of extracellular vesicles was harvested from U251MG cells stably transduced with retroviral expression vectors encoding ACE2 and the corresponding second part of the split protein of the label fused to CD63. Defined aliquots of supernatant harvested directly from the transfected 293T cell culture containing the donor type of extracellular vesicles were used and incubated with serial dilutions of serum for 30 min as indicated. The recipient type of extracellular vesicles was purified and concentrated from supernatant of U251MG cells. Defined aliquots of the recipient type of extracellular vesicles were added to the serum dilutions containing the donor type of extracellular vesicles followed by an incubation period of 4 hours. After adding substrate to the mixture, the results were obtained with the aid of a luminometer.

Characteristic neutralization curves with sera from two donors were established with the methodology described in the description of Figure 3. Serum of a naïve donor showed no neutralization even at high concentrations (see Figure 3A). Serum of an individual after having received a SARS-CoV-2 vaccine (see Figure 3B) showed high neutralization. The extent of neutralization, termed titer of neutralizing antibodies, was determined by calculating the dilution at which 50 % neutralization occurred. VLPN = Virus-like particle neutralization.

### Example 4:

Validation of the novel Virus-like particle neutralization test (VLPNT), comparing 23 sera from COVID-19 patients analyzed in the VLPNT was conducted (see Figure 4). The identical sera were also analyzed using the conventional Virus neutralization test (cVNT) in a BSL3 laboratory with replication competent SARS-CoV-2 virus in a plaque reduction assay. The results from both tests correlated very well according to statistical analyses provided.

### Example 5:

An adaption of the Virus-like particle neutralization test was conducted (see Figure 5) with two variants of concern (VOC) of SARS-CoV-2 (Wuhan D614G and B.1.617.2) and neutralizing serum titers as in Example 4, but with sera from vaccinees being analyzed and compared. The VLPNT could be easily modified to analyze the sera using the predominant B.1.617.2 mutant of the Spike protein of current SARS-CoV-2 field isolates and compare the results with the Wuhan D614G variant of Spike. In line with the literature, neutralizing antibody titers of vaccinees were less potent in neutralizing the SARS-CoV-2 δ-mutant (B.1.617.2) compared with the Wuhan D614G Spike protein.

### Example 6:

Results with sera from a naïve and a vaccinated donor analyzed in the cell-free virus neutralization test with two types of extracellular vesicles are shown in Figure 6. The y-axis depicts the relative amount (percentage) of light units using a split label approach with internal test standards to delineate the range of the assay (0 and 100 %). The x-axis shows serum dilutions. Serum of the individual who has received a SARS-CoV-2 vaccine (see Figure 3B) showed high titers of neutralization antibodies.

### REFERENCE

Manuel Albanese, Yen-Fu Adam Chen, Corinna Hüls, Kathrin Gärtner, Takanobu Tagawa, Oliver T. Keppler, Christine Göbel, Reinhard Zeidler and Wolfgang Hammerschmidt, Micro RNAs are minor constituents of extracellular vesicles and are hardly delivered to target cells, bioRxiv print doi.org/10.1101/2020.05.20.106393.
Hu J. et al.: "Development of cell-based pseudovirus entry assay to identify potential viral entry inhibitors and neutralizing antibodies against SARS-CoV-2", GENES & DISEASES, vol. 7, no. 4, 17 July 2020, pages 551-557.
Saeed M. F. et al.: "Novel, rapid assay for measuring entry of diverse enveloped viruses, including HIV and rabies", JOURNAL OF VIROLOGICAL METHODS, vol. 135, no. 2, 2006, pages 143-150.
Spitzer D. et al.: "Green Fluorescent Protein-Tagged Retroviral Envelope Protein for Analysis of Virus-Cell Interactions", JOURNAL OF VIROLOGY, vol. 77, no. 10, 2003, pages 6070-6075.
Tscherne D. M. et al.: "An enzymatic virus-like particle assay for sensitive detection of virus entry", JOURNAL OF VIROLOGICAL METHODS, vol. 163, no. 2, 2010, pages 336-343.
Wolf M. C. et al.: "A catalytically and genetically optimized 12-lactamase-matrix based assay for sensitive, specific, and higher throughput analysis of native henipavirus entry characteristics", VIROLOGY JOURNAL, BIOMED CENTRAL, vol. 6, no. 1, 2009, page 119.
Zettl F. et al.: "Quantification of SARS-CoV-2-Neutralizing Antibodies Using Propagation-Defective Vesicular Stomatitis Virus Pseudotypes", VACCINES, vol. 8, no. 3, 2020, page 386.

## Claims

1. A method for determining whether or not virus-neutralizing antibodies are present in a sample obtained from a subject, comprising the following steps:
- providing extracellular vesicles and a label, wherein the extracellular vesicles
(i) are non-infectious,
(ii) comprise one or more viral glycoprotein(s),
(iii) are detectable via the label,
(iv) are free of recombinant genetic material, and
(v) comprise extracellular vesicle proteins that are incorporated into the membrane or the envelope of the extracellular vesicles, wherein a membrane protein in the extracellular vesicles is CD63;
- contacting the sample with said extracellular vesicles and cells, which are capable of taking up said extracellular vesicles, wherein the one or more viral glycoprotein(s) is/ are able to target a receptor of said cells and is/ are fusogenic, and
- determining whether or not said cells take up said extracellular vesicles;
wherein a reduced uptake of said extracellular vesicles by said cells in comparison to a control, wherein said cells and said extracellular vesicles are contacted, but without said sample, is indicative of the presence of virus-neutralizing antibodies.

2. The method of claim 1,
wherein the label is a protein selected from the group consisting of beta-galactosidase, beta-lactamase, beta-glucuronidase, a luciferase and any combination thereof, and/or
wherein the receptor is selected from the group consisting of angiotensin converting enzyme 2 (ACE2), CD46, CD150 (signaling lymphocyte-activation molecule SLAM), lysosomal associated membrane protein 1 (LAMP1), T-cell immunoglobin mucin domain-1 (TIM-1), sialyl-(alpha-2,3)-galactosidase-receptor, sialyl-(alpha-2,6)-galactosidase-receptor, CD21 and a MHC class II receptor.

3. The method of claim 1 or 2,
wherein the label is a split protein, wherein a first part of the split protein is comprised in the extracellular vesicles and a second part of the split protein is comprised in the cells, and
wherein the first and the second part are able to form a complex,
preferably wherein the first part of the split protein, which is comprised in the extracellular vesicles, is attached to:
(i) an extracellular vesicle protein of the extracellular vesicles, or
(ii) a viral capsid-protein or a viral nucleo-protein of the extracellular vesicles, or
(iii) a viral tegument protein of the extracellular vesicles, or
(iv) the one or more viral glycoprotein(s) of the extracellular vesicles.

4. The method of claim 1 or 2,
wherein the extracellular vesicles are detectable via the label,
wherein the label is attached to an extracellular vesicle protein comprised in the extracellular vesicles,
preferably wherein the extracellular vesicles comprise the extracellular vesicle protein and the label as a fusion protein, and/or
preferably wherein the extracellular vesicle protein is selected from the group consisting of a tetraspanin;
more preferably CD63, CD40, CD81, CD9, CD37, CD53, CD54, CD151, CD82 and TSPAN-8;
an integrin;
more preferably alpha-3, alpha-5, alpha-V, alpha-6, beta-1 or beta-3 integrin; and a type I membrane protein, or
preferably wherein the extracellular vesicle protein is a viral extracellular vesicle protein.

5. The method of claim 1 or 2,
wherein the extracellular vesicles are detectable via the label,
wherein the label is attached to a viral capsid-protein or a viral nucleo-protein and
wherein the one or more viral glycoprotein(s) and the viral capsid-protein or the viral nucleo-protein are from the same virus.

6. The method of claim 5,
wherein the viral capsid-protein is selected from the group consisting of HIV-1, and a viral capsid-protein of a herpes virus, or
wherein the viral nucleo-protein is selected from the group consisting of a viral nucleo-protein from an Epstein-Barr virus, a measles virus, an influenza virus, a parainfluenza virus, a human respiratory syncytial virus, an Ebola virus, a Marburg virus, a hanta virus, a Lassa virus and the nucleo-protein N of a SARS-CoV-virus, preferably the nucleo-protein N of the SARS-CoV-2-virus.

7. The method of claim 1 or 2,
wherein the extracellular vesicles are detectable via the label,
wherein the label is attached to the one or more viral glycoprotein(s) of the extracellular vesicles,
preferably wherein the one or more viral glycoprotein(s) is/ are not the M-, E- or S-protein of the SARS-CoV-2-virus.

8. The method of any one of the previous claims,
wherein the virus-neutralizing antibodies are neutralizing antibodies against a virus selected from the group consisting of a coronavirus, Epstein-Barr virus, measles virus, influenza virus, parainfluenza virus, human respiratory syncytial virus, Ebola virus, hanta virus and Lassa virus, and/or
wherein the one or more viral glycoprotein(s) is/ are on the surface of the extracellular vesicles, and/or
wherein the one or more viral glycoprotein(s) is/ are one or more viral glycoprotein(s) from a virus selected from the group consisting of a coronavirus, an Epstein-Barr virus, measles virus, influenza virus, parainfluenza virus, human respiratory syncytial virus, Ebola virus, hanta virus and Lassa virus, and/or
wherein the extracellular vesicles are detectable via the label, wherein the label is attached to a viral tegument protein of the extracellular vesicles, and wherein the one or more viral glycoprotein(s) and the viral tegument protein are from the same virus.

9. The method of any one of the previous claims,
wherein said cell is a cell selected from the group consisting of a primary cell, a cell line, an epithelial cell, an immune cell and a cell line engineered to express a viral vector,
preferably a viral receptor selected from the group consisting of angiotensin converting enzyme 2 (ACE2), CD46, CD150 (signaling lymphocyte-activation molecule SLAM), lysosomal associated membrane protein 1 (LAMP1), T-cell immunoglobin mucin domain-1 (TIM-1), sialyl-(alpha-2,3)-galactosidase-receptor, sialyl-(alpha-2,6)-galactosidase-receptor, CD21 and a MHC class II receptor.

10. A method for determining whether or not virus-neutralizing antibodies are present in a sample obtained from a subject, comprising the following steps:
- providing a donor type of extracellular vesicles and a label,
wherein the donor type of extracellular vesicles
(i) are non-infectious,
(ii) comprise one or more viral glycoprotein(s),
(iii) are detectable via the label,
(iv) are free of recombinant genetic material, and
(v) comprise proteins that are incorporated into the membrane or the envelope of the extracellular vesicles, wherein a membrane protein in the extracellular vesicles is CD63;
- contacting the sample with said donor type of extracellular vesicles and a recipient type of extracellular vesicles, which are capable of taking up said donor type of extracellular vesicles, and
- determining whether or not said recipient type of extracellular vesicles take up said donor type of extracellular vesicles;
wherein a reduced uptake of said donor type of extracellular vesicles by said recipient type of extracellular vesicles in comparison to a control, wherein said donor type of extracellular vesicles and said recipient type of extracellular vesicles are contacted, but without said sample, is indicative of the presence of virus-neutralizing antibodies.

11. The method according to claim 10,
wherein the label is a split protein,
wherein a first part of the split protein is comprised in the donor type of extracellular vesicles and a second part of the split protein is comprised in the recipient type of extracellular vesicles, and
wherein the first and the second part of the split protein are able to form a complex.

12. An *in vitro* method for screening (a) compound(s) for its/ their ability to neutralize a virus, comprising the following steps:
- providing extracellular vesicles and a label,
wherein the extracellular vesicles
(i) are non-infectious,
(ii) comprise one or more viral glycoprotein(s),
(iii) are detectable via the label,
(iv) are free of recombinant genetic material, and
(v) comprise proteins that are incorporated into the membrane or the envelope of the extracellular vesicles, wherein a membrane protein in the extracellular vesicles is CD63;
- contacting said compound(s) with said extracellular vesicles and cells, which are capable of taking up said extracellular vesicles, wherein the one or more viral glycoprotein(s) is/ are able to target a receptor of said cells and is/ are fusogenic, and
- determining whether or not said cells take up said extracellular vesicles;
wherein a reduced uptake of said extracellular vesicles by said cells in comparison to a control, wherein said cells and said extracellular vesicles are contacted, but without said compound(s), is indicative of the ability of said compound(s) to neutralize said virus.

13. The *in vitro* method according to claim 12, wherein the label is a split protein,
wherein a first part of the split protein is comprised in the extracellular vesicles and a second part of the split protein is comprised in the cells, and
wherein the first and the second part of the split protein are able to form a complex.

14. An *in vitro* method for screening (a) compound(s) for its/ their ability to neutralize a virus, comprising the following steps:
- providing a donor type of extracellular vesicles and a label,
wherein the extracellular vesicles
(i) are non-infectious,
(ii) comprise one or more viral glycoprotein(s),
(iii) are detectable via the label,
(iv) are free of recombinant genetic material, and
(v) comprise proteins that are incorporated into the membrane or the envelope of the extracellular vesicles, wherein a membrane protein in the extracellular vesicles is CD63;
- contacting said compound(s) with said donor type of extracellular vesicles and a recipient type of extracellular vesicles, which are capable of taking up said donor type of extracellular vesicles, and
- determining whether or not said recipient type of extracellular vesicles take up said donor type of extracellular vesicles;
wherein a reduced uptake of said donor type of extracellular vesicles by said recipient type of extracellular vesicles in comparison to a control, wherein said donor type of extracellular vesicles and said recipient type of extracellular vesicles are contacted, but without said compound(s), is indicative of the ability of said compound(s) to neutralize said virus.

15. The *in vitro* method according to claim 14,
wherein the label is a split protein,
wherein a first part of the split protein is comprised in the donor type of extracellular vesicles and a second part of the split protein is comprised in the recipient type of extracellular vesicles, and
wherein the first and the second part of the split protein are able to form a complex.

## Patentansprüche

1. Verfahren zum Bestimmen, ob virusneutralisierende Antikörper in einer von einem Subjekt entnommenen Probe vorhanden sind, umfassend die folgenden Schritte:
- Bereitstellen von extrazellulären Vesikeln und einem Label, wobei die extrazellulären Vesikel
(i) nicht-infektiös sind,
(ii) ein virales Glykoprotein oder mehrere virale Glykoproteine umfassen,
(iii) über das Label nachweisbar sind,
(iv) frei von rekombinantem genetischem Material sind und
(v) extrazelluläre Vesikelproteine umfassen, die in die Membran oder die Hülle der extrazellulären Vesikel eingebaut sind, wobei ein Membranprotein in den extrazellulären Vesikeln CD63 ist;
- Inkontaktbringen der Probe mit den extrazellulären Vesikeln und Zellen, die in der Lage sind, die extrazellulären Vesikel aufzunehmen, wobei das eine virale Glykoprotein oder die mehreren viralen Glykoproteine in der Lage ist/ sind, auf einen Rezeptor dieser Zellen abzuzielen und fusogen ist/sind, und
- Bestimmen, ob diese Zellen die extrazellulären Vesikel aufnehmen oder nicht;
wobei eine verringerte Aufnahme dieser extrazellulären Vesikel durch diese Zellen im Vergleich zu einer Kontrolle, bei der diese Zellen und diese extrazellulären Vesikel in Kontakt gebracht werden, jedoch ohne die Probe, auf das Vorhandensein von virusneutralisierenden Antikörpern hinweist.

2. Verfahren nach Anspruch 1,
wobei das Label ein Protein ist, das aus der Gruppe ausgewählt ist, die aus Beta-Galactosidase, Beta-Lactamase, Beta-Glucuronidase, einer Luciferase und einer beliebigen Kombination davon besteht, und/oder
wobei der Rezeptor ausgewählt ist aus der Gruppe bestehend aus Angiotensin-konvertierendem Enzym 2 (ACE2), CD46, CD150 (Signal-Lymphozyten-Aktivierungsmolekül SLAM), Lysosomen assoziiertem Membranprotein 1 (LAMP1), T-Zell-Immunglobulin-Mucin-Domäne-1 (TIM-1), Sialyl-(alpha-2,3)-Galactosidase-Rezeptor, Sialyl-(alpha-2,6)-Galactosidase-Rezeptor, CD21 und einem MHC-Klasse-II-Rezeptor.

3. Verfahren nach Anspruch 1 oder 2,
wobei das Label ein gespaltenes Protein ist, wobei ein erster Teil des gespaltenen Proteins in den extrazellulären Vesikeln enthalten ist und ein zweiter Teil des gespaltenen Proteins in den Zellen enthalten ist, und
wobei der erste und der zweite Teil in der Lage sind, einen Komplex zu bilden,
bevorzugt wobei der erste Teil des gespaltenen Proteins, der in den extrazellulären Vesikeln enthalten ist, gebunden ist an:
(i) ein extrazelluläres Vesikelprotein der extrazellulären Vesikel oder
(ii) ein virales Kapsidprotein oder ein virales Nukleoprotein der extrazellulären Vesikel oder
(iii) ein virales Tegumentprotein der extrazellulären Vesikel oder
(iv) ein virales Glykoprotein oder mehrere virale Glykoproteine der extrazellulären Vesikel.

4. Verfahren nach Anspruch 1 oder 2,
wobei die extrazellulären Vesikel über das Label nachweisbar sind,
wobei das Label an ein extrazelluläres Vesikelprotein gebunden ist, das in den extrazellulären Vesikeln enthalten ist,
bevorzugt wobei die extrazellulären Vesikel das extrazelluläre Vesikelprotein und das Label als Fusionsprotein umfassen, und/oder
bevorzugt wobei das extrazelluläre Vesikelprotein aus der Gruppe ausgewählt ist, die aus einem Tetraspanin; bevorzugt CD63, CD40, CD81, CD9, CD37, CD53, CD54, CD151, CD82 und TSPAN-8; einem Integrin; bevorzugt Alpha-3-, Alpha-5-, Alpha-V-, Alpha-6-, Beta-1- oder Beta-3-Integrin; und einem Typ-I-Membranprotein besteht, oder
bevorzugt wobei das extrazelluläre Vesikelprotein ein virales extrazelluläres Vesikelprotein ist.

5. Verfahren nach Anspruch 1 oder 2,
wobei die extrazellulären Vesikel über das Label nachweisbar sind,
wobei das Label an ein virales Kapsidprotein oder ein virales Nukleoprotein gebunden ist und
wobei das eine virale Glykoprotein oder die mehreren viralen Glykoproteine und das virale Kapsidprotein oder das virale Nukleoprotein aus demselben Virus stammen.

6. Verfahren nach Anspruch 5,
wobei das virale Kapsidprotein aus der Gruppe ausgewählt ist, die aus HIV-1 und einem viralen Kapsidprotein eines Herpesvirus besteht, oder
wobei das virale Nukleoprotein aus der Gruppe ausgewählt ist, die aus einem viralen Nukleoprotein eines Epstein-Barr-Virus, eines Masernvirus, eines Influenzavirus, eines Parainfluenzavirus, eines humanen respiratorischen Synzytialvirus, eines Ebola-Virus, eines Marburg-Virus, eines Hanta-Virus, eines Lassa-Virus und dem Nukleoprotein N eines SARS-CoV-Virus, bevorzugt dem Nukleoprotein N des SARS-CoV-2-Virus, besteht.

7. Verfahren nach Anspruch 1 oder 2,
wobei die extrazellulären Vesikel über das Label nachweisbar sind,
wobei das Label an dem einen viralen Glykoprotein oder den mehreren viralen Glykoproteinen der extrazellulären Vesikel angebracht ist,
bevorzugt wobei das eine virale Glykoprotein oder die mehreren viralen Glykoproteine nicht das M-, **E-** oder S-Protein des SARS-CoV-2-Virus sind.

8. Verfahren nach irgendeinem der vorhergehenden Ansprüche,
wobei die virusneutralisierenden Antikörper neutralisierende Antikörper gegen ein Virus sind, das aus der Gruppe ausgewählt ist, die aus einem Coronavirus, einem Epstein-Barr-Virus, einem Masernvirus, einem Influenzavirus, einem Parainfluenzavirus, einem humanen respiratorischen Synzytialvirus, einem Ebolavirus, einem Hantavirus und einem Lassavirus besteht, und/oder
wobei das eine virale Glykoprotein oder die mehreren viralen Glykoproteine auf der Oberfläche der extrazellulären Vesikel vorhanden ist/sind, und/oder
wobei das eine virale Glykoprotein oder die mehreren viralen Glykoproteine ein virales Glykoprotein oder mehrere virale Glykoproteine aus einem Virus ist/sind, das aus der Gruppe ausgewählt ist, die aus einem Coronavirus, einem Epstein-Barr-Virus, einem Masernvirus, einem Influenzavirus, einem Parainfluenzavirus, einem humanen respiratorischen Synzytialvirus, einem Ebolavirus, einem Hantavirus und einem Lassavirus besteht, und/oder
wobei die extrazellulären Vesikel über das Label nachweisbar sind, wobei das Label an ein virales Tegumentprotein der extrazellulären Vesikel gebunden ist und wobei das eine virale Glykoprotein oder die mehreren viralen Glykoproteine und das virale Tegumentprotein aus demselben Virus stammen.

9. Verfahren nach irgendeinem der vorhergehenden Ansprüche,
wobei die Zelle eine Zelle ist, die aus der Gruppe ausgewählt ist, die aus einer Primärzelle, einer Zelllinie, einer Epithelzelle, einer Immunzelle und einer Zelllinie besteht, die so konstruiert ist, dass sie einen viralen Vektor exprimiert,
bevorzugt ein viraler Rezeptor, ausgewählt aus der Gruppe bestehend aus Angiotensin-konvertierendem Enzym 2 (ACE2), CD46, CD150 (Signal-Lymphozyten-Aktivierungsmolekül SLAM), Lysosomen assoziiertem Membranprotein 1 (LAMP1), T-Zell-Immunglobulin-Mucin-Domäne-1 (TIM-1), Sialyl-(alpha-2,3)-Galactosidase-Rezeptor, Sialyl-(alpha-2,6)-Galactosidase-Rezeptor, CD21 und einem MHC-Klasse-II-Rezeptor.

10. Verfahren zum Bestimmen, ob virusneutralisierende Antikörper in einer von einem Subjekt gewonnenen Probe vorhanden sind, umfassend die folgenden Schritte:
- Bereitstellen eines Spendertyps von extrazellulären Vesikeln und ein Label,
wobei die extrazellulären Vesikel vom Spendertyp
(i) nicht-infektiös sind,
(ii) ein virales Glykoprotein oder mehrere virale Glykoproteine umfassen,
(iii) über das Label nachweisbar sind,
(iv) frei von rekombinantem genetischem Material sind und
(v) Proteine umfassen, die in die Membran oder die Hülle der extrazellulären Vesikel eingebaut sind, wobei ein Membranprotein in den extrazellulären Vesikeln CD63 ist;
- Inkontaktbringen der Probe mit dem Spendertyp von extrazellulären Vesikeln und einem Empfängertyp extrazellulärer Vesikel, die in der Lage sind, den Spendertyp von extrazellulären Vesikeln aufzunehmen, und
- Bestimmen, ob die extrazellulären Vesikel des Empfängertyps die extrazellulären Vesikel des Spendertyps aufnehmen oder nicht;
wobei eine verringerte Aufnahme extrazellulärer Vesikel des Spendertyps durch extrazelluläre Vesikel des Empfängertyps im Vergleich zu einer Kontrolle, bei der extrazelluläre Vesikel des Spendertyps und extrazelluläre Vesikel des Empfängertyps in Kontakt gebracht werden, jedoch ohne die Probe, auf das Vorhandensein von virusneutralisierenden Antikörpern hinweist.

11. Verfahren nach Anspruch 10,
wobei das Label ein gespaltenes Protein ist,
wobei ein erster Teil des gespaltenen Proteins in dem Spendertyp extrazellulärer Vesikel enthalten ist und ein zweiter Teil des gespaltenen Proteins in dem Empfängertyp extrazellulärer Vesikel enthalten ist, und
wobei der erste und der zweite Teil des gespaltenen Proteins in der Lage sind, einen Komplex zu bilden.

12. In-vitro-Verfahren zum Screenen von (einer) Verbindung(en) auf ihre Fähigkeit, ein Virus zu neutralisieren, umfassend die folgenden Schritte:
- Bereitstellen von extrazellulären Vesikeln und einem Label,
wobei die extrazellulären Vesikel
(i) nicht-infektiös sind,
(ii) ein virales Glykoprotein oder mehrere virale Glykoproteine umfassen,
(iii) über ein Label nachweisbar sind,
(iv) frei von rekombinantem, genetischem Material sind und
(v) Proteine umfassen, die in die Membran oder die Hülle der extrazellulären Vesikel eingebaut sind, wobei ein Membranprotein in den extrazellulären Vesikeln CD63 ist;
- Inkontaktbringen dieser Verbindung(en) mit den extrazellulären Vesikeln und Zellen, die in der Lage sind, diese extrazellulären Vesikel aufzunehmen, wobei das eine virale Glykoprotein oder die mehreren viralen Glykoproteine in der Lage ist/sind, auf einen Rezeptor dieser Zellen abzuzielen und fusogen ist/sind, und
- Bestimmen, ob die Zellen die extrazellulären Vesikel aufnehmen oder nicht;
wobei eine verringerte Aufnahme der extrazellulären Vesikel durch diese Zellen im Vergleich zu einer Kontrolle, bei der diese Zellen und diese extrazellulären Vesikel in Kontakt gebracht werden, jedoch ohne die Verbindung(en), ein Hinweis auf die Fähigkeit der Verbindung(en) ist, das Virus zu neutralisieren.

13. In-vitro-Verfahren nach Anspruch 12, wobei das Label ein gespaltenes Protein ist,
wobei ein erster Teil des gespaltenen Proteins in den extrazellulären Vesikeln enthalten ist und ein zweiter Teil des gespaltenen Proteins in den Zellen enthalten ist, und
wobei der erste und der zweite Teil des gespaltenen Proteins in der Lage sind, einen Komplex zu bilden.

14. In-vitro-Verfahren zum Screening von (einer) Verbindung(en) hinsichtlich ihrer Fähigkeit, ein Virus zu neutralisieren, umfassend die folgenden Schritte:
- Bereitstellen eines Spendertyps extrazellulärer Vesikel und eines Label,
wobei die extrazellulären Vesikel
(i) nicht-infektiös sind,
(ii) ein virales Glykoprotein oder mehrere virale Glykoproteine umfassen,
(iii) über das Label nachweisbar sind,
(iv) frei von rekombinantem, genetischem Material sind und
(v) Proteine umfassen, die in die Membran oder die Hülle der extrazellulären Vesikel eingebaut sind, wobei ein Membranprotein in den extrazellulären Vesikeln CD63 ist;
- Inkontaktbringen der Verbindung(en) mit dem Spendertyp extrazellulärer Vesikel und einem Empfängertyp extrazellulärer Vesikel, die in der Lage sind, den Spendertyp extrazellulärer Vesikel aufzunehmen, und
- Bestimmen, ob die extrazellulären Vesikel des Empfängertyps die extrazellulären Vesikel des Spendertyps aufnehmen oder nicht;
wobei eine verringerte Aufnahme extrazellulärer Vesikel des Spendertyps durch extrazelluläre Vesikel des Empfängertyps im Vergleich zu einer Kontrolle, bei jener Spendertyp von extrazellulären Vesikeln und jener Empfängertyp von extrazellulären Vesikeln in Kontakt gebracht werden, jedoch ohne die Verbindung(en), ein Hinweis auf die Fähigkeit der Verbindung(en) ist, das Virus zu neutralisieren.

15. Das *In-vitro-Verfahren* gemäß Anspruch **14,**
wobei das Label ein gespaltenes Protein ist,
wobei ein erster Teil des gespaltenen Proteins in dem Spendertyp extrazellulärer Vesikel enthalten ist und ein zweiter Teil des gespaltenen Proteins in dem Empfängertyp extrazellulärer Vesikel enthalten ist, und
wobei der erste und der zweite Teil des gespaltenen Proteins in der Lage sind, einen Komplex zu bilden.

## Revendications

1. Procédé permettant de déterminer si des anticorps neutralisant un virus sont présents dans un échantillon prélevé sur un sujet, comprenant les étapes suivantes:
- fournir des vésicules extracellulaires et un marqueur, les vésicules extracellulaires
(i) non infectieuses,
(ii) comprennent une ou plusieurs glycoprotéines virales,
(iii) sont détectables par le marqueur,
(iv) sont exemptes de matériel génétique recombinant et
(v) comprennent des protéines vésiculaires extracellulaires incorporées dans la membrane ou l'enveloppe des vésicules extracellulaires, une protéine membranaire dans les vésicules extracellulaires étant CD63;
- mettre en contact l'échantillon avec les vésicules extracellulaires et les cellules capables d'absorber les vésicules extracellulaires, dans lequel la ou les glycoprotéines virales sont capables de cibler un récepteur des cellules et sont fusogènes, et
- déterminer si les cellules absorbent ou non les vésicules extracellulaires;
une absorption réduite des vésicules extracellulaires par les cellules par rapport à un contrôle dans lequel les cellules et les vésicules extracellulaires sont mises en contact, mais sans l'échantillon, indiquant la présence d'anticorps neutralisant le virus.

2. Procédé selon la revendication 1,
dans lequel le marqueur est une protéine choisie dans le groupe constitué par la bêta-galactosidase, la bêta-lactamase, la bêta-glucuronidase, une luciférase et toute combinaison de celles-ci, et/ou
le récepteur étant choisi dans le groupe constitué par l'enzyme de conversion de l'angiotensine 2 (ACE2), CD46, CD150 (molécule d'activation des lymphocytes SLAM), la protéine membranaire associée aux lysosomes 1 (LAMP1), le domaine 1 de la mucine immunoglobuline des cellules T (TIM-1), le récepteur de la sialyl-(alpha-2,3)-galactosidase, le récepteur de la sialyl-(alpha-2,6)-galactosidase, le CD21 et un récepteur MHC de classe **II.**

3. Procédé selon la revendication 1 ou 2,
dans lequel le marqueur est une protéine clivée, une première partie de la protéine clivée étant contenue dans les vésicules extracellulaires et une deuxième partie de la protéine clivée étant contenue dans les cellules, et
les première et deuxième parties étant capables de former un complexe,
de préférence dans lequel la première partie de la protéine clivée contenue dans les vésicules extracellulaires est liée à:
(i) une protéine extracellulaire des vésicules extracellulaires, ou
(ii) une protéine de capside virale ou une nucléoprotéine virale des vésicules extracellulaires, ou
(iii) une protéine tégumentaire virale des vésicules extracellulaires ou
(iv) une ou plusieurs glycoprotéines virales des vésicules extracellulaires.

4. Procédé selon la revendication 1 ou 2,
dans lequel les vésicules extracellulaires sont détectables via le marqueur,
le marqueur étant lié à une protéine de vésicule extracellulaire contenue dans les vésicules extracellulaires,
de préférence les vésicules extracellulaires comprenant la protéine de vésicule extracellulaire et le marqueur sous forme de protéine de fusion, et/ou
de préférence, la protéine vésiculaire extracellulaire étant choisie dans le groupe constitué par une tétraspanine; de préférence CD63, CD40, CD81, CD9, CD37, CD53, CD54, CD151, CD82 et TSPAN-8; une intégrine; de préférence une intégrine alpha-3, alpha-5, alpha-V, alpha-6, bêta-1 ou bêta-3; et une protéine membranaire de type I, ou
de préférence, la protéine vésiculaire extracellulaire étant une protéine vésiculaire extracellulaire virale.

5. Procédé selon la revendication 1 ou 2,
dans lequel les vésicules extracellulaires sont détectables via le marqueur,
le marqueur étant lié à une protéine de capside virale ou à une nucléoprotéine virale, et
dans lequel la ou les glycoprotéines virales et la protéine de capside virale ou la nucléoprotéine virale proviennent du même virus.

6. Procédé selon la revendication 5,
dans lequel la protéine de capside virale est choisie dans le groupe constitué par le VIH-1 et une protéine de capside virale d'un virus de l'herpès, ou
dans lequel la nucléoprotéine virale est choisie dans le groupe constitué par une nucléoprotéine virale d'un virus d'Epstein-Barr, d'un virus de la rougeole, d'un virus de la grippe, d'un virus parainfluenza, d'un virus respiratoire syncytial humain, d'un virus Ebola, d'un virus Marburg, d'un virus Hanta, d'un virus Lassa et de la nucléoprotéine N d'un virus SARS-CoV, de préférence la nucléoprotéine N du virus SARS-CoV-2.

7. Procédé selon la revendication 1 ou 2,
dans lequel les vésicules extracellulaires sont détectables par le marquage,
le marquage étant apposé sur la ou les glycoprotéines virales des vésicules extracellulaires,
de préférence, dans lequel la ou les glycoprotéines virales ne sont pas la protéine M, E ou S du virus SARS-CoV-2.

8. Procédé selon l'une des revendications précédentes,
dans lequel les anticorps neutralisant le virus sont des anticorps neutralisant un virus choisi dans le groupe constitué par un coronavirus, le virus d'Epstein-Barr, le virus de la rougeole, le virus de la grippe, le virus parainfluenza, le virus respiratoire syncytial humain, le virus Ebola, le virus Hanta et le virus Lassa, et/ou
dans lequel la ou les glycoprotéines virales sont présentes à la surface des vésicules extracellulaires, et/ou
dans lequel la ou les glycoprotéines virales sont une ou plusieurs glycoprotéines virales provenant d'un virus choisi dans le groupe constitué par un coronavirus, un virus d'Epstein-Barr, un virus de la rougeole, un virus de la grippe, un virus parainfluenza, un virus respiratoire syncytial humain, un virus Ebola, un virus Hanta et un virus Lassa, et/ou
les vésicules extracellulaires étant détectables par le marqueur, le marqueur étant lié à une protéine tégumentaire virale des vésicules extracellulaires, et la ou les glycoprotéines virales et la protéine tégumentaire virale provenant du même virus.

9. Procédé selon l'une des revendications précédentes,
dans lequel la cellule est une cellule choisie dans le groupe constitué d'une cellule primaire, d'une lignée cellulaire, d'une cellule épithéliale, d'une cellule immunitaire et d'une lignée cellulaire conçue pour exprimer un vecteur viral,
de préférence un récepteur viral choisi dans le groupe constitué par l'enzyme de conversion de l'angiotensine 2 (ACE2), CD46, CD150 (molécule d'activation des lymphocytes SLAM), la protéine membranaire associée aux lysosomes 1 (LAMP1), le domaine mucine immunoglobuline des cellules T 1 (TIM-1), le récepteur de la sialyl-(alpha-2,3)-galactosidase, le récepteur de la sialyl-(alpha-2,6)-galactosidase, le CD21 et un récepteur MHC de classe II.

10. Procédé permettant de déterminer si des anticorps neutralisant un virus sont présents dans un échantillon prélevé sur un sujet, comprenant les étapes suivantes:
- Fournir un type de donneur de vésicules extracellulaires et un marqueur,
les vésicules extracellulaires du type donneur
(i) ne sont pas infectieuses,
(ii) comprennent une ou plusieurs glycoprotéines virales,
(iii) sont détectables via le marqueur,
(iv) sont exemptes de matériel génétique recombinant et
(v) comprennent des protéines incorporées dans la membrane ou l'enveloppe des vésicules extracellulaires, une protéine membranaire dans les vésicules extracellulaires étant CD63;
- mettre en contact l'échantillon avec le type de vésicules extracellulaires du donneur et un type de vésicules extracellulaires du receveur capables d'absorber le type de vésicules extracellulaires du donneur, et
- déterminer si les vésicules extracellulaires de type récepteur absorbent ou non les vésicules extracellulaires de type donneur;
une absorption réduite des vésicules extracellulaires de type donneur par les vésicules extracellulaires de type receveur par rapport à un contrôle dans lequel les vésicules extracellulaires de type donneur et les vésicules extracellulaires de type receveur sont mises en contact, mais sans l'échantillon, indiquant la présence d'anticorps neutralisant le virus.

11. Procédé selon la revendication 10,
dans lequel le marqueur est une protéine clivée,
une première partie de la protéine clivée étant contenue dans le type de vésicule extracellulaire donneur et une deuxième partie de la protéine clivée étant contenue dans le type de vésicule extracellulaire receveur, et
les première et deuxième parties de la protéine clivée étant capables de former un complexe.

12. Procédé in vitro pour cribler un ou plusieurs composés quant à leur capacité à neutraliser un virus, comprenant les étapes suivantes:
- la fourniture de vésicules extracellulaires et d'un marqueur,
les vésicules extracellulaires
(i) non infectieuses,
(ii) comprennent une ou plusieurs glycoprotéines virales,
(iii) sont détectables par le marqueur,
(iv) sont exemptes de matériel génétique recombinant et
(v) comprennent des protéines intégrées dans la membrane ou l'enveloppe des vésicules extracellulaires, une protéine membranaire dans les vésicules extracellulaires étant CD63;
- mettre en contact le ou les composés avec les vésicules extracellulaires et les cellules capables d'absorber les vésicules extracellulaires, dans lesquelles la ou les glycoprotéines virales sont capables de cibler un récepteur des cellules et sont fusogènes, et
- Déterminer si les cellules absorbent ou non les vésicules extracellulaires;
une absorption réduite des vésicules extracellulaires par les cellules par rapport à un contrôle dans lequel les cellules et les vésicules extracellulaires sont mises en contact, mais sans le ou les composés, étant une indication de la capacité du ou des composés à neutraliser le virus.

13. Procédé in vitro selon la revendication 12, dans lequel le marqueur est une protéine clivée,
une première partie de la protéine clivée étant contenue dans les vésicules extracellulaires et une deuxième partie de la protéine clivée étant contenue dans les cellules, et
les première et deuxième parties de la protéine clivée étant capables de former un complexe.

14. Procédé in vitro pour cribler (a) des composés en fonction de leur capacité à neutraliser un virus, comprenant les étapes suivantes:
- la fourniture d'un type de vésicules extracellulaires donneuses et d'un marqueur,
les vésicules extracellulaires
(i) non infectieuses,
(ii) comprennent une ou plusieurs glycoprotéines virales,
(iii) sont détectables par le marqueur,
(iv) sont exemptes de matériel génétique recombinant et
(v) comprennent des protéines intégrées dans la membrane ou l'enveloppe des vésicules extracellulaires, une protéine membranaire dans les vésicules extracellulaires étant CD63;
- mettre en contact le ou les composés avec le type de vésicules extracellulaires donneuses et un type de vésicules extracellulaires receveuses capables de recevoir le type de vésicules extracellulaires donneuses, et
- Déterminer si les vésicules extracellulaires de type receveur absorbent ou non les vésicules extracellulaires de type donneur;
une absorption réduite du type de vésicules extracellulaires donneuses par le type de vésicules extracellulaires receveuses par rapport à un contrôle dans lequel le type de vésicules extracellulaires donneuses et le type de vésicules extracellulaires receveuses sont mis en contact, mais sans le ou les composés, étant une indication de la capacité du ou des composés à neutraliser le virus.

15. Procédé *in vitro* selon la revendication 14,
dans lequel le marqueur est une protéine clivée,
une première partie de la protéine clivée étant contenue dans le type de vésicule extracellulaire donneur et une deuxième partie de la protéine clivée étant contenue dans le type de vésicule extracellulaire receveur, et
dans lequel les première et deuxième parties de la protéine clivée sont capables de former un complexe.
